# EUROPEAN PATENT SPECIFICATION

(11) **EP 4 178 588 B1**
(45) Date of publication and mention of the grant of the patent: **07.05.2025**
(21) Application number: 21837202.7
(22) Date of filing: 07.07.2021
(51) Int. Cl.: A61K 33/40, A61K 33/22, A61P 35/00, C01B 15/12, C12N 15/63

(54) **ANTI-CANCER ACTIVITY OF PERBORATE SALTS**
ANTIKREBSWIRKUNG VON PERBORATSALZEN
ACTIVITÉ ANTICANCÉREUSE DE SELS DE PERBORATE

(30) Priority: 09.07.2020 WO PCT/CA2020/050950
(43) Date of publication of application: 17.05.2023
(73) Proprietor: Aliya Pharmaceuticals Inc., Toronto ON M2P 2H1 (CA)
(72) Inventor: DALAN, Altay Burak, Toronto, Ontario M2P 2H1 (CA); SAHIN, Fikrettin, 34755 Atasehir - Istanbul (TR); CEBECI, Emre, 34755 Atasehir - Istanbul (TR); BAYRAK, Ömer Faruk, 34755 Atasehir - Istanbul (TR)
(74) Representative: Gill Jennings & Every LLP
(86) International application number: PCT/CA2021/050932
(87) International publication number: WO 2022/006673

(56) References cited:
- WO-A2-2006/102439
- WO-A2-2006/102439
- GB-A- 2 215 205
- US-A1- 2007 048 389
- US-A1- 2008 008 769
- WADE T. BARRANCO ET AL: "Boric acid inhibits human prostate cancer cell proliferation", CANCER LETTERS, vol. 216, no. 1, 1 December 2004 (2004-12-01), pages 21 - 29, XP055146289, ISSN: 0304-3835, DOI: 10.1016/j.canlet.2004.06.001
- CEBECI EMRE ET AL: "Anti-cancer effect of boron derivatives on small-cell lung cancer", JOURNAL OF TRACE ELEMENTS IN MEDICINE AND BIOLOGY, FISCHER, NEW YORK, NY, US, vol. 70, 4 January 2022 (2022-01-04), XP086931386, ISSN: 0946-672X, [retrieved on 20220104], DOI: 10.1016/J.JTEMB.2022.126923
- OMEROGLU ULU ZEHRA ET AL: "Integrated transcriptome and in vitro analysis revealed anti-proliferative effect of sodium perborate on hepatocellular carcinoma cells", JOURNAL OF TRACE ELEMENTS IN MEDICINE AND BIOLOGY, FISCHER, NEW YORK, NY, US, vol. 73, 3 June 2022 (2022-06-03), XP087136091, ISSN: 0946-672X, [retrieved on 20220603], DOI: 10.1016/J.JTEMB.2022.127011

## Description

### FIELD

This disclosure relates to the field of anti-cancer therapy. In particular, the disclosure relates to perborate salts, compositions comprising the same, and uses thereof as anti-cancer agents.

### BACKGROUND

Cancer is the general name of various diseases that result in the continuous growth and division of cells in the body due to the disruption of control mechanisms. Ten properties that distinguish cancer cells from normal cells include: the ability to synthesize the growth factors they need, immunity to signals that restrict their growth, the ability to escape controlled cell death pathways, the ability to divide unlimitedly, the ability to form a blood vessel network that can feed themselves, the ability to metastasize and invade other tissues and organs through blood vessels, immunity to the ability to escape the system, increase tumor inflammation, irregular metabolic pathways, and an unstable genome that is open to mutations (Hanahan & Weinberg, 2011).

Cancer is the second most fatal disease after cardiovascular diseases, with lung cancer causing the most deaths amongst all cancer types. Surgical, radiotherapeutic and/or chemotherapeutic methods are commonly used as cancer treatments, each having variable levels of efficacy depending on the type of cancer.

For treatments that use chemotherapy, even the most effective chemotherapy drugs are not able to eliminate cancer completely, and merely serve to extend the patient's life. Furthermore, chemotherapy drugs do not solely affect cancer cells. Chemotherapy drugs may be targeted to cancer cells, e.g. by using cancer's interest in sugar, but this does not prevent entry to healthy cells. As a result, chemotherapies have many side effects such as emesis, vertigo, weight loss, fever, decreased blood values and bleeding. These side effects are only some of the 23 known side effects of 250 chemotherapy drugs supported by the NCI (National Cancer Institute). In addition, the side effects of existing chemotherapeutic drugs vary from patient to patient.

Thus, there remains a need for alternative treatments for treating various cancers in order to eliminate the cancer, or extend a patient's life.

Boric acid is known for use in the treatment of certain types of cancer: WADE T. BARRANCO ET AL: "Boric acid inhibits human prostate cancer cell proliferation",CANCER LETTERS, vol. 216, no. 1, 1 December 2004 (2004-12-01), pages 21-29.

GB 2 215 205 A discloses perborate salts for use in medicine, in particular for the treatment of acne and bromidrosis.

WO 2006/102439 A2 discloses the use of perborate salts as an oxidising agent in chemotherapy compositions.

### SUMMARY

The present invention discloses a perborate salt for use in the treatment of cancer. The invention is defined by the claims.

In one aspect, the present disclosure provides a perborate salt for use in a method for reducing gene expression of one or more genes in a cancer cell or a tumor cell, the method comprising contacting the cell with an effective amount of a perborate salt, wherein the one or more genes are selected from the group consisting of Paxillin (PXN), Vinculin (VCL), TLN1, TNS2, RHOA, CDC42, KRAS, CCN2, C-Met and BIRC5.

In some embodiments, the one or more genes is PXN. In some embodiments, the one or more genes is VCL. In some embodiments, the one or more genes is TLN1. In some embodiments, the one or more genes is TNS2. In some embodiments, the one or more genes is RHOA. In some embodiments, the one or more genes is CDC42. In some embodiments, the one or more genes is KRAS. In some embodiments, the one or more genes is CCN2. In some embodiments, the one or more genes is C-met. In some embodiments, the one or more genes is BIRC5 (survivin).

In various embodiments, the cell is characterized by upregulation of the EGF/EGFR pathway as compared to normal cells. In some embodiments, the cancer or tumor cell is a bladder cancer cell, a bone cancer cell, a brain tumor cell, a breast cancer cell, a cervical cancer cell, a colorectal cancer cell, an endometrial cancer cell, a gastric cancer (adenocarcinoma) cell, a leukemia cell, a liver cancer cell, a lung cancer cell, a lymphoma cell, an oral cancer cell, an ovarian cancer cell, a pancreatic cancer cell, a prostate cancer cell, a renal cancer cell, a skin cancer cell or a thyroid cancer cell. In some embodiments, the cancer or tumor cell is a lung cancer cell, a pancreatic cancer cell, a chordoma cell, a breast cancer cell or an adenocarcinoma cell. In some embodiments, the cancer or tumor cell is a lung cancer cell. In some embodiments, the cancer or tumor cell is a pancreatic cancer cell. In some embodiments, the cancer or tumor cell is a chordoma cell.

In another aspect, the present disclosure provides a pharmaceutical composition comprising a perborate salt and a pharmaceutically acceptable carrier, diluent or adjuvant. The pharmaceutical composition may comprise an effective amount of a perborate salt. The pharmaceutical composition may further comprise a chemotherapy agent, such as Cisplatin and/or 5-fluorouracil.

In another aspect, the present disclosure provides a perborate salt for use in the treatment of a cancer. For example, an effective amount of a perborate salt for use in the treatment of cancer. The present disclosure further provides a combination comprising an effective amount of a perborate salt and a chemotherapy agent, such as Cisplatin and/or 5-fluorouracil.

In another aspect, the present disclosure provides a perborate salt for use in a method for treating a cancer in a subject, the method comprising administering a perborate salt to the subject. For example, the method comprises administering an effective amount of a perborate salt to the subject. The method may further comprise administering a chemotherapy agent, such as Cisplatin and/or 5-fluorouracil, to the subject.

In another aspect, the present disclosure provides a pharmaceutical composition comprising an effective amount of a perborate salt and a pharmaceutically acceptable carrier, diluent or adjuvant.

In another aspect, the present disclosure provides an effective amount of a perborate salt for use in the treatment of a cancer. The cancer may be a lung cancer, a pancreatic cancer, a chordoma, a breast cancer or an adenocarcinoma. For example, the cancer may be small cell lung cancer. Also provide is a combination comprising the effective amount of the perborate salt and a chemotherapy agent.

In another aspect, the present disclosure provides use of a perborate salt for treatment of a cancer in a subject. For example, use of an effective amount of a perborate salt for treatment of a cancer in a subject. The perborate salt may be in combination with a chemotherapy agent, such as Cisplatin and/or 5-fluorouracil.

In another aspect, the present disclosure provides use of a perborate salt in preparation of a medicament for treatment of a cancer in a subject. For example, use of an effective amount of a perborate salt in preparation of a medicament for treatment of a cancer in a subject. The perborate salt may be in combination with a chemotherapy agent, such as Cisplatin and/or 5-fluorouracil.

In some embodiments, the perborate salt may be in anhydrous form or solvent addition form. The perborate salts may be associated with a non-stoichiometric amount of a solvent, water and/or buffer. The solvent may be, for example, a pharmaceutically acceptable solvent. In some embodiments, the perborate salt may have the general formula XBO₃·nH₂O (n = 1 to 6). In some embodiments, the perborate salt is a tetrahydrate perborate salt.

The perborate salt may be a group 1 or group 2 metal salt (e.g. where X is a group 1 or group 2 metal ion). In some embodiments, the perborate salt may be a group 1 metal salt. In some embodiments, the perborate salt may be a sodium perborate salt, a potassium perborate salt or a lithium perborate salt. In some embodiments, the perborate salt is a sodium perborate salt, e.g. having the formula NaBO₃·nH₂O (n = 1 to 6). In some embodiments, the perborate salt is sodium perborate tetrahydrate.

In various embodiments, the cancer is characterized by upregulation of the EGF/EGFR pathway as compared to normal cells. In various embodiments, the cancer is a bladder cancer, a bone cancer, a brain tumor, a breast cancer, a cervical cancer, a colorectal cancer, an endometrial cancer, a gastric cancer (adenocarcinoma), a chordoma, a liver cancer, a lung cancer, a lymphoma, an oral cancer, an ovarian cancer, a pancreatic cancer, a prostate cancer, a renal cancer, a skin cancer, a thyroid cancer or a leukemia. In some embodiments, the cancer is a lung cancer, a pancreatic cancer, a chordoma, a breast cancer or an adenocarcinoma. In some embodiments, the cancer is a lung cancer, such as small cell lung cancer. In some embodiments, the cancer is a pancreatic cancer. In some embodiments, the cancer is a chordoma.

The perborate salt may be provided at a dosage of at least about 0.05 mg/kg/day. The perborate salt may be provided at a dosage of between about 0.05 mg/kg/day to about 10 mg/kg/day. The perborate salt may be provided at a dosage of up to about 115 mg/kg/day.

In another aspect, the present disclosure provides a perborate salt for use in a method for reducing gene expression of one or more genes in a cancer cell or a tumor cell, the method comprising contacting the cell with an effective amount of a perborate salt, wherein the one or more genes are involved in a cell signalling pathway selected from the group consisting of: cell cycle pathway, DNA replication pathway, EGF/EGFR signal pathway, Focal Adhesion-PI3K-AKT pathway, focal adhesion pathway, G protein signaling pathway, Hippo-Merlin Signaling dysregulation pathway, MAPK signaling pathway, actin-cytoskeleton pathway, TGF Beta signaling pathway and WNT signaling pathway.

The one or more genes may be a gene involved in the cell cycle pathway, such as one or more genes selected from the group consisting of: ANAPC5, ANAPC11, CCNA2, CCNB1, CCNB2, CCNB3, CCND3, CCNE1, CCNE2, CDC20, CDC23, CDC25A, CDC25B, CDC25C, CDC45, CDC6, CDC7, CDK1, CDK2, CDK4, CDKN2A, CDKN2C, CUL1, E2F1, E2F2, E2F3, HDAC1, HDAC2, MCM2, MCM3, MCM4, MCM5, MCM6, MCM7, MYC, ORC1, ORC6, PCNA, PLK1, PTTG1, RAD21, RBL1, RBX1, BUB1, BUB3, SKP2, SMC1A, STAG1, TFDP1, TGFB1, TTK, YWHAB, and YWHAG.

The one or more genes may be a gene involved in the DNA replication pathway, such as one or more genes selected from the group consisting of: CDC6, CDC7, CDK2, CDT1, GMNN, MCM10, MCM2, MCM3, MCM4, MCM5, MCM6, MCM7, PCNA, POLA2, POLD2, POLD3, POLE, POLE2, RFC2, RFC3, RFC4, RPA2, RPA3, and PRIM1.

The one or more genes may be a gene involved in the EGF/EGFR pathway, such as one or more genes selected from the group consisting of: AP2S1, ARHGEF1, AURKA, E2F1, INPP5D, MYBL2, NEDD4, PCNA, PEBP1, PIK3R1, PLCE1, PRKCB, RAF1, RALB, RALBP1, RPS6KA1, RPS6KA3, SHC1, and STMN1.

The one or more genes may be a gene involved in the focal adhesion pathway, such as one or more genes selected from the group consisting of: ANGPT2, CDC37, COL3A1, EIF4E, EIF4E2, ELAVL1, EPHA2, EPOR, FGF18, FGF2, FGFR1, GNB2, GNG4, GNG10, GNG12, HSP90AA1, HSP90AB1, HSP90B1, IKBKG, IRS1, ITGB3, LAMA1, LAMB1, LAMC3, PIK3R1, PPP2CA, PPP2CB, PPP2R1A, PPP2R1B, PPP2R3C, RAB11B, RAF1, SLC2A3, TBC1D1, THBS1, ITGB3, LAMA1, LAMB1, THBS1, LAMC3, SHC1, SHC4, STYK1, PRKCB, ACTN1, ACTN4, FLNA, ZYX, VASP, VCL, PARVB, PARVA, RHOA, PIK3R1, PPP1CA, PPP1CC, MYL9, MYL12B, MYLK, ACTB, ACTG1, PAK4, CCND3, BIRC3, and RAF1.

The one or more genes may be a gene involved in the G protein pathway, such as one or more genes selected from the group consisting of: AKAP5, AKAP8, AKAP9, GNB2, GNG4, GNG10, GNG12, GNAI2, PDE7A, PRKAR1B, RRAS, CALM1, GNA12, ARHGEF1, RHOA, PRKCH, PRKD3, and PRKCQ.

The one or more genes may be selected from a gene involved in the Hippo pathway, such as one or more genes selected from the group consisting of: AJUBA, CD44, EPHA2, FGFR1, FOXM1, ITGB3, LATS2, MYC, NF2, PAK4, PPP1CA, PPP1CC, PRKAR1B, RBX1, and TEAD4.

The one or more genes may be a gene involved in the MAPK/ERK pathway such as one or more genes selected from the group consisting of: ARRB1, CASP3, CDC25B, DUSP6, DUSP9, ECSIT, FGF18, FGF2, FGFR1, FLNA, GNA12, HSPA1A, HSPA1B, HSPA2, HSPA8, IKBKG, MAP2K3, MAP2K6, MAP2K7, MAP3K11, MAPK11, MYC, NFKB1, PLA2G4E, PPP5C, PPP5D1, RAF1, RASGRP1, RPS6KA3, RRAS, STMN1, and TGFB1.

The one or more genes may be a gene involved in the actin cytoskeleton pathway, such as one or more genes selected from the group consisting of: ACTB, ACTG1, ACTN1, ARHGEF1, ARPC5, FGF18, FGF2, FGFR1, GNG12, MSN, MYH10, MYLK, PAK4, PIK3R1, RAF1, RHOA, RRAS, TMSB4X, and VCL.

The one or more genes may be a gene involved in the TGF beta signal pathway, such as one or more genes selected from the group consisting of: CCNB2, CDK1, CITED1, CUL1, EID2, FOXP3, HDAC1, ITGB3, MAP2K6, MMP1, MYC, PARD6A, PIK3R1, RAF1, RBL1, RBX1, RHOA, SHC1, SIK1, TERT, TFDP1, TGFB1, TGFB1I1, THBS1, TRAP1, WWP1, and ZEB2.

The one or more genes may be a gene involved in the WNF signal pathway, such as one or more genes selected from the group consisting of: CAMK2G, CCND3, CTBP1, CTNNB1, GPC4, LRP6, MYC, PLAU, PRICKLE1, PRKCB, RHOA, ROR2, SFRP1, VANGL1, WNT5A, and WNT5B.

The chemotherapy agent may comprise Altretamine; Bendamustine; Busulfan; Carboplatin; Carmustine; Chlorambucil; Cisplatin; Cyclophosphamide; Dacarbazine; Ifosfamide; Lomustine; Mechlorethamine; Chlormethine; Melphalan; Oxaliplatin; Temozolomide; Thiotepa; Trabectedin; Carmustine; Lomustine; Streptozocin; Azacitidine; 5-fluorouracil (5-FU); 6-mercaptopurine (6-MP); Capecitabine (Xeloda); Cladribine; Clofarabine; Cytarabine (Ara-C); Decitabine; Floxuridine; Fludarabine; Gemcitabine (Gemzar); Hydroxyurea; Methotrexate; Nelarabine; Pemetrexed (Alimta); Pentostatin; Pralatrexate; Thioguanine; Trifluridine/tipiracil combination; Daunorubicin; Doxorubicin; Epirubicin; Idarubicin; Valrubicin; Bleomycin; Dactinomycin; Mitomycin-C; Mitoxantrone; Irinotecan; Topotecan; Etoposide (VP-16); Mitoxantrone; Teniposide; Cabazitaxel; Docetaxel; Nab-paclitaxel; Paclitaxel; Vinblastine; Vincristine; Vinorelbine; Prednisone; Methylprednisolone; Dexamethasone; Arsenic trioxide; Asparaginase; Eribulin; Hydroxyurea; Ixabepilone; Mitotane; Omacetaxine; Pegaspargase; Procarbazine; Romidepsin; Vorinostat, Raltitrexed or any combinations thereof. In some embodiments, the chemotherapy agent comprises Cisplatin. In some embodiments, the chemotherapy agent comprises 5-fluorouracil.

Other aspects and features of the present invention will become apparent to those of ordinary skill in the art upon review of the following description of specific embodiments of the invention in conjunction with the accompanying claims.

### BRIEF DESCRIPTION OF THE DRAWINGS

In drawings which illustrate embodiments of the invention,
**Figure 1** shows the effect of sodium perborate tetrahydrate on death pathways of DMS114 cells (small cell lung cancer cells). NC: negative control, SPT: sodium perborate tetrahydrate.
**Figure 2** shows the effect of sodium perborate tetrahydrate on death pathways of MRC5 cells (healthy lung cells). NC: negative control, SPT: sodium perborate tetrahydrate.
**Figures 3A** and **3B** show the effect of sodium perborate tetrahydrate on DMS114 and MRC5 cell survival.
**Figures 4A-4F** show the effects of sodium perborate tetrahydrate on chordoma (CH22, JHC7, UM-Chor1, MUG-Chor1, U-CH1,) and normal (NPH) cell survival.
**Figures 5A-5C** show the effects of sodium perborate tetrahydrate on pancreatic cancer (PSN1, BxPC3 and ASPC) cell survival.
**Figure 6** shows the effect of sodium perborate tetrahydrate on Paxillin, Vinculin and Talin-1 gene expression levels in DMS114 cell line. NC: negative control, SPT: sodium perborate tetrahydrate.
**Figure 7** shows the effect of sodium perborate tetrahydrate on RhoA and Cdc42 gene expression levels in DMS114 cell line. NC: negative control, SPT: sodium perborate tetrahydrate.
**Figure 8** shows the effect of the sodium perborate tetrahydrate treatment on the expression levels of proliferation genes CYR61, MKI67, PCNA, CTGF, E2F4 and TP53 in MRC-5 cell line. The left hand side bar for each gene is NC: negative control and the right hand side bar for each gene is SPT: sodium perborate tetrahydrate treatment.
**Figures 9A** and **9B** show the effect of sodium perborate tetrahydrate on KRAS, CCN2, C-Met and BIRC5 gene expression levels in DMS114 cell line, and BIRC5 gene expression levels in MRC5 cell line. NC: negative control, SPT: sodium perborate tetrahydrate.
**Figures 10A** and **10B** together are a schematic diagram showing the cell cycle pathway. Genes whose expression level is reduced two-fold or more are shown in shaded grey boxes. The bold wavy lines indicate that the diagram is split over Figures 10A and 10B.
**Figure 11** is a schematic diagram showing the DNA Replication pathway. Genes whose expression level is reduced two-fold or more are shown in shaded grey boxes.
**Figures 12A** and **12** **B** together are a schematic diagram showing the EGF / EGFR signal pathway. Genes whose expression level is reduced two-fold or more are shown in shaded grey boxes. The bold wavy lines indicate that the diagram is split over Figures 12A and 12B.
**Figures 13A** and **13B** together are a schematic diagram showing the Focal Adhesion-Pl3K-AKT pathway. Genes whose expression level is reduced two-fold or more are shown in shaded grey boxes. The bold wavy lines indicate that the diagram is split over Figures 13A and 13B.
**Figures 14A** and **14B** together are a schematic diagram showing the Focal Adhesion pathway. Genes whose expression level is reduced two-fold or more are shown in shaded grey boxes. The bold wavy lines indicate that the diagram is split over Figures 14A and 14B.
**Figure 15** is a schematic diagram showing the G protein signaling pathway. Genes whose expression level is reduced two-fold or more are shown in shaded grey boxes.
**Figure 16** is a schematic diagram showing the Hippo-Merlin Signaling dysregulation pathway. Genes whose expression level is reduced two-fold or more are shown in shaded grey boxes.
**Figures 17A** and **17B** together are a schematic diagram showing the MAPK signaling pathway. Genes whose expression level is reduced two-fold or more are shown in shaded grey boxes. The bold wavy lines indicate that the diagram is split over Figures 17A and 17B.
**Figure 18A** and **18B** together are a schematic diagram showing regulation of actin cytoskeleton pathway. Genes whose expression level is reduced two-fold or more are shown in shaded grey boxes. The bold wavy lines indicate that the diagram is split over Figures 18A and 18B.
**Figures 19A** and **19B** together are a schematic diagram showing the TGF Beta Signaling pathway. Genes whose expression level is reduced two-fold or more are shown in shaded grey boxes. The bold wavy lines indicate that the diagram is split over Figures 19A and 19B.
**Figures 20A** and **20B** together are a schematic diagram showing the WNT Signaling pathway. Genes whose expression level is reduced two-fold or more are shown in shaded grey boxes. The bold wavy lines indicate that the diagram is split over Figures 20A and 20B.
**Figures 21A** and **21****B** are SEM images of DMS114 cells at the start of treatment with sodium perborate tetrahydrate and after 3 days treatment, respectively.
**Figures 22A** and **22B** are SEM images of MRC5 cells at the start of treatment with sodium perborate tetrahydrate and after 3 days treatment, respectively.
**Figures 23A-C** show the cell viability of small-cell lung cancer cells (DMS114) 24 hours, 48 hours and 72 hours after treatment with three different boron derivatives: boric acid (Figure 23A), sodium pentaborate pentahydrate (Figure 23B) and sodium perborate tetrahydrate (Figure 23C).
**Figure 24** shows the effect of sodium perborate tetrahydrate on cell survival of PSN-1 (a pancreatic adenocarcinoma cell line) 24h, 48h and 72h after treatment. The EC50 concentration is 7.5 µg/mL. NC: negative control, SPT: sodium perborate tetrahydrate.
**Figure 25** shows the effect of cisplatin on PSN-1 cell survival 24h, 48h and 72h after treatment. The EC50 concentration is 2 mM. NC: negative control.
**Figure 26** shows the effect of 5-fluorouracil (5-FU) on PSN-1 cell survival 24h, 48h and 72h after treatment. The EC50 concentration is 13 mM. NC: negative control, SPT: sodium perborate tetrahydrate.
**Figure 27** shows the effect of a combination of sodium perborate tetrahydrate (4 µg/mL) and cisplatin on PSN-1 cell survival 24h, 48h and 72h after treatment. The EC50 concentration of 5-FU is 1 mM. TNC: negative control, SPT: sodium perborate tetrahydrate.
**Figure 28** shows the effect of a combination of sodium perborate tetrahydrate (4 µg/mL) and 5-fluorouracil (5-FU) on PSN-1 cell survival 24h, 48h and 72h after treatment. The EC50 concentration of 5-FU is 6.5 mM. NC: negative control, SPT: sodium perborate tetrahydrate.

### DETAILED DESCRIPTION

In the context of the present disclosure, various terms are used in accordance with what is understood to be the ordinary meaning of those terms.

This disclosure is based in part on the discovery that perborate salts may be used for reducing or decreasing gene expression of one or more genes in a cancer cell or a tumor cell. The one or more genes may be selected from the group consisting of PXN, VCL, TLN1, TNS2, RHOA, CDC42, KRAS, CCN2, C-Met and BIRC5. The one or more genes may be involved in a cell signalling pathway, the cell signalling pathway being a cell cycle pathway, a DNA replication pathway, an EGF/EGFR signal pathway, a Focal Adhesion-Pl3K-AKT pathway, a focal adhesion pathway, a G protein signaling pathway, a Hippo-Merlin Signaling dysregulation pathway, a MAPK signaling pathway, an actin-cytoskeleton pathway, a TGF Beta signaling pathway or a WNT signaling pathway. In various embodiments, the perborate salts as described herein may be used in the preparation of a medicament or a composition for treatment of a cancer.

Further aspects of the disclosure make use of compositions comprising a perborate salt and a pharmaceutically acceptable carrier, diluent or adjuvant. A perborate salt for use in methods of treating a cancer are also provided. Such methods may include administering a perborate salt or a composition of a perborate salt, or an effective amount of a perborate salt or a composition of a perborate salt, to a subject in need thereof. The compositions, and uses comprising perborate salts as described herein may be in combination with one or more chemotherapy agent.

### Perborate salts

The term "perborate salt" refers to a compound of the general formula XBO₃·nH₂O, where X is a metal element and n is an integer from 0 to 6. For the hydrated perborate salt, n is an integer from 1 to 6.

In some embodiments, the metal element may be a group 1 metal element, such as lithium, sodium, potassium, rubidium, cesium or francium. For example, the metal element may be lithium, sodium or potassium. In some embodiments, the metal element may be sodium.

In alternative embodiments, the metal element may be a group 2 metal element such as magnesium, calcium, strontium, barium or radium.

The perborate salt may be anhydrous (e.g. XBO₃). The perborate salt may be a monohydrate, dihydrate, trihydrate, or tetrahydrate. In some embodiments, the perborate salt is a monohydrate salt. In some embodiments, the perborate salt is a dihydrate salt. In some embodiments, the perborate salt is a trihydrate salt. In some embodiments, the perborate salt is a tetrahydrate salt. In some embodiments, the perborate salt is a solvate.

The perborate salts contain a perborate anion [(B(OH)₂OO)₂]²⁻ consisting of a -B-O-O-B-O-O- core with two hydroxyl groups attached to each boron atom. The embodiments of perborate salts as described include all possible stereochemical alternatives, including those illustrated or described herein. For example, the ring may adopt a chair conformation - see Formula (la):

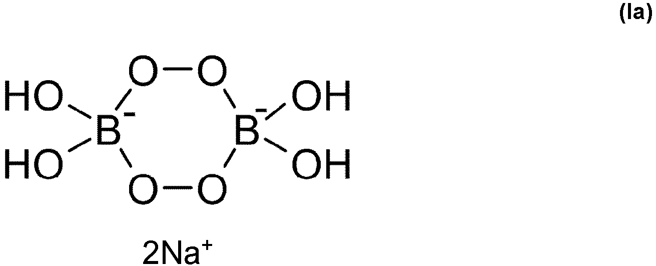

Sodium perborate may be manufactured by reaction of borax Na₂B₄O₇ and sodium hydroxide (NaOH) to give sodium metaborate NaBO₂, which may then be reacted with hydrogen peroxide to give hydrated sodium perborate.

The raw material cost of the perborate salts (tetrahydrate and others) is approximately USD$350 per ton. The raw materials required to synthesise perborate salts are abundant in nature and they are readily synthesized in comparison to other cancer drugs. Perborate salts are stable and have a relatively long shelf life compared to other chemotherapy drugs. They are suitable candidates for incorporating in drug delivery systems which may be designed to target 'hard to reach' cancer cells, e.g. the perborate salts can readily be coated with polyethylene glycol or be loaded in liposomes or other types of target delivery vehicles such as nanoparticles.

While the present disclosure exemplifies the use of sodium perborate as an anti-cancer agent, it is known in the art that other perborate salts have similar physical properties and therefore are expected to have similar physiological and pharmokinetic properties.

### Toxicity of perborates

The perborates have a long history of safe use in bleach-containing cleaning products, e.g. laundry detergents and machine dishwashing detergents, and are known to have low toxicity via oral, inhalation and dermal exposure routes in humans. Furthermore, sodium perborate solutions have been classified as not cytotoxic (Masetti, 2018).

Boron itself is a mineral found in foods such as nuts and is commonly taken as a medical supplement for conditions such as boron deficiency, menstrual cramps and osteoarthritis. Boron regulates calcium, potassium and magnesium metabolism in the body, and is associated with proper bone growth and development (Dessordi, 2017; Meacham, 1995). Boron is also a known food preservative. It has been shown that taking up to 18 mg of boron daily in adults does not cause any side effects (Murray, 1995). It has also been shown that boron homeostasis in mammalian systems is excreted through the urinary tract within 96 hours (Toxicological Review Of Boron And Compounds, CAS No. 7440-42-8).

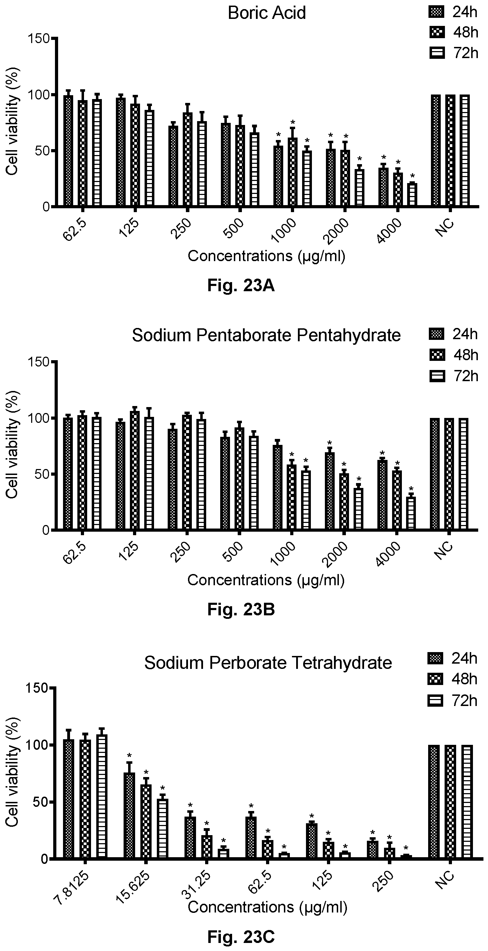

While perborates are precursors of boric acid in the body, the biological effects and toxicology profiles of boric acid and other boron derivatives are noticeably different for both animals and humans (Biatek, 2019; Reproductive and General Toxicology of some Inorganic Borates and Risk Assessment for Human Beings, 1995; SCCS/1249/09, 2010; SCCS/1345/10, 2010). The difference in biological activity is confirmed by Figures 23A-C (Example 7) indicating that in the same biological model, sodium perborate tetrahydrate is significantly less toxic than boric acid and sodium pentaborate pentahydrate.

In the human body, sodium perborate tetrahydrate undergoes multiple reactions. For example, sodium perborate tetrahydrate may dissociate into hydrogen peroxide, sodium metaborate and water (see Scheme I), or hydrogen peroxide and sodium borate (borax). Hydrogen peroxide further degrades to water and oxygen. However, NMR and Raman spectroscopy indicate that in dilute solution, an equilibrium exists that still contains peroxoborate anions and that these peroxoborate species are able to deliver the hydroperoxide anion at a lower pH than when H₂O₂ is used (Scheme II; A. McKillop, W. R. Sanderson, Tetrahedron, 1995, 51, 6145-6166). Furthermore, some borates are not metabolized at all, and the fact that perborates can be detected in urine indicates that some perborates are not converted to boric acid in the body (Reproductive and General Toxicology of some Inorganic Borates and Risk Assessment for Human Beings, 1995; Large-scale human metabolic phenotyping and molecular epidemiological studies via 1H NMR spectroscopy of urine investigation of borate preservation - PMID: 19453167).

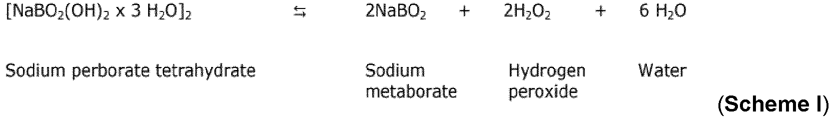

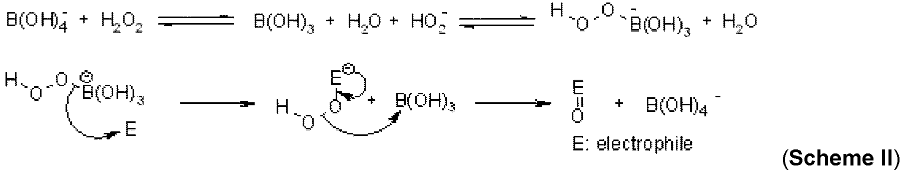

Sodium perborate also reacts with acetic acid in the body (Scheme III; A. McKillop, W. R. Sanderson, Tetrahedron, 1995, 51, 6145-6166).

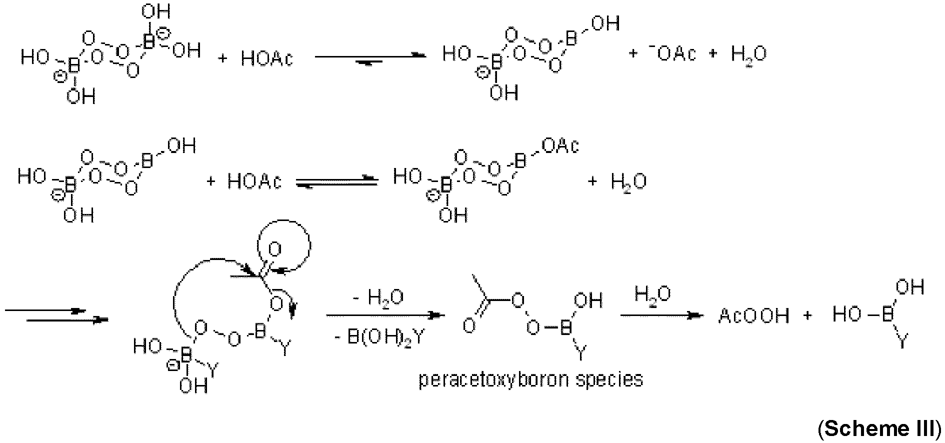

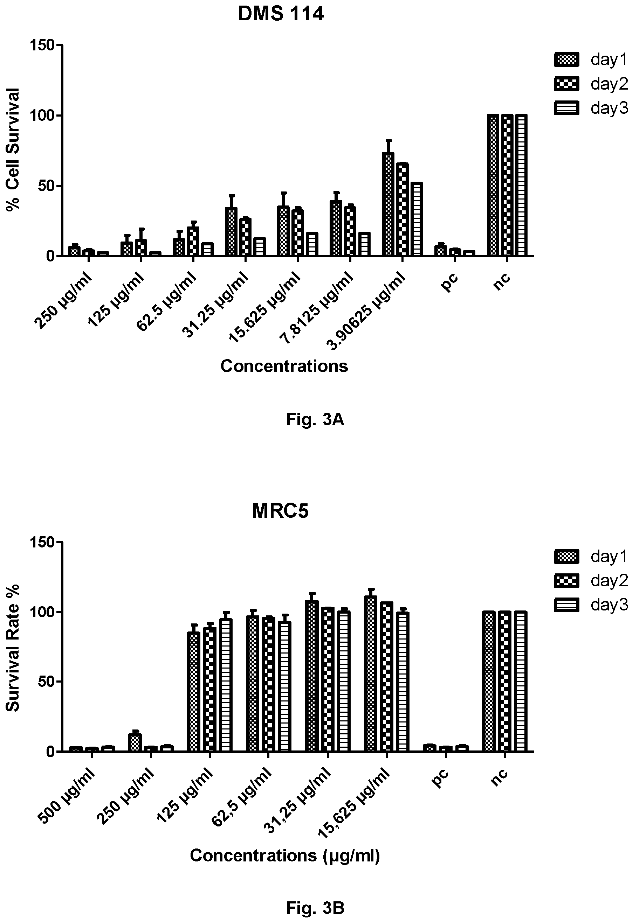

As shown in Figure 3A, sodium perborate tetrahydrate shows the maximum effect on DMS114 cells within 1 day. More specifically, video analysis of DMS114 cells treated with sodium perborate tetrahydrate showed that the maximum effect on cell survival was seen within 18 hours (see Example 3). In this way, the excess amount of the perborate salts used in cancer treatment will be easily removed from the body after anti-cancer activity has been achieved. The video analysis also showed that sodium perborate tetrahydrate stopped the movement of DMS114 cells close to 100%, thereby preventing possible metastasis of the cancer cell.

Video analysis of MRC5 cells treated with sodium perborate tetrahydrate also showed that the damage to healthy cells only slowed the division and movement of these cells, and these effects disappeared when the boron moves away from the environment (see Example 3). Figure 3B shows that the perborate salts do not cause permanent damage to the healthy cells at concentrations between 125 µg/mL and 625 µg/mL.

Toxicology studies performed in rats also confirmed that sodium perborate tetrahydrate had no significant toxicity to healthy tissues (see Table 2).

### Anti-cancer activity

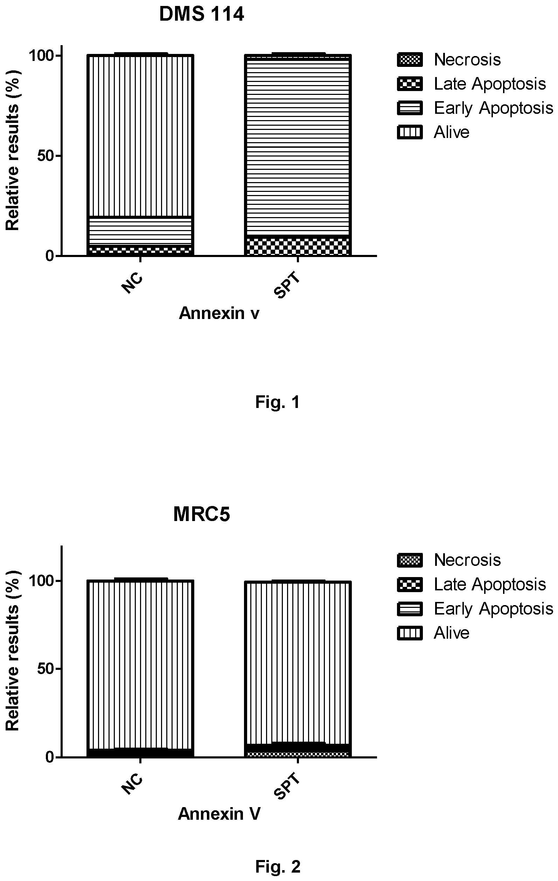

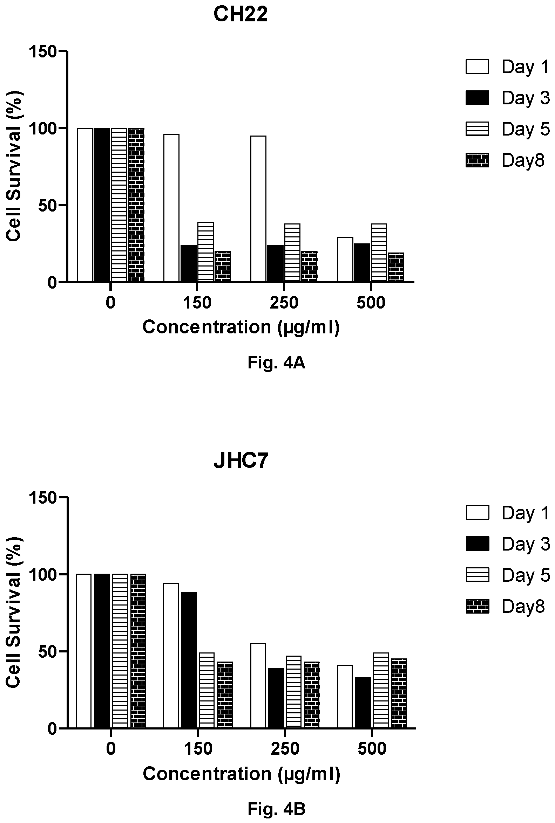

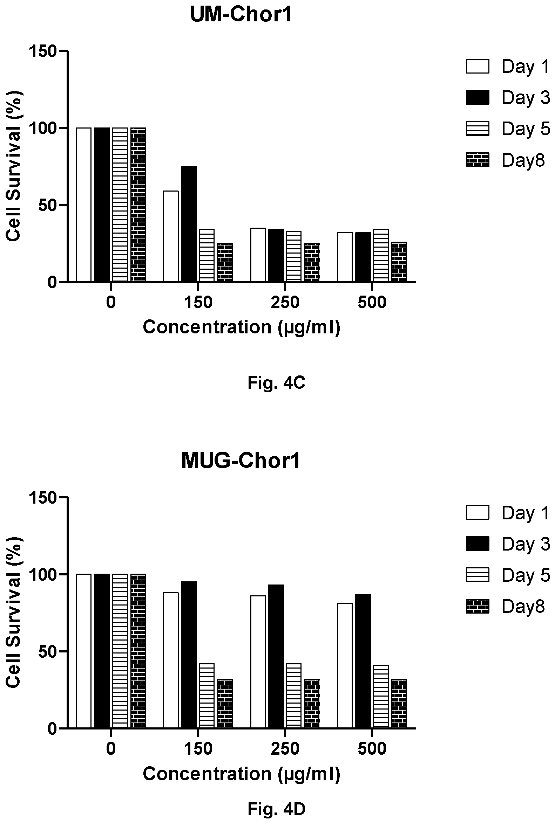

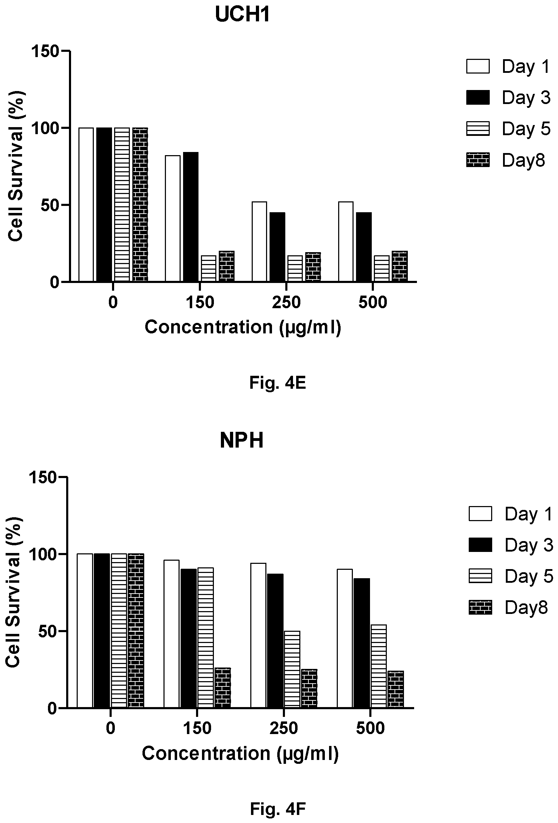

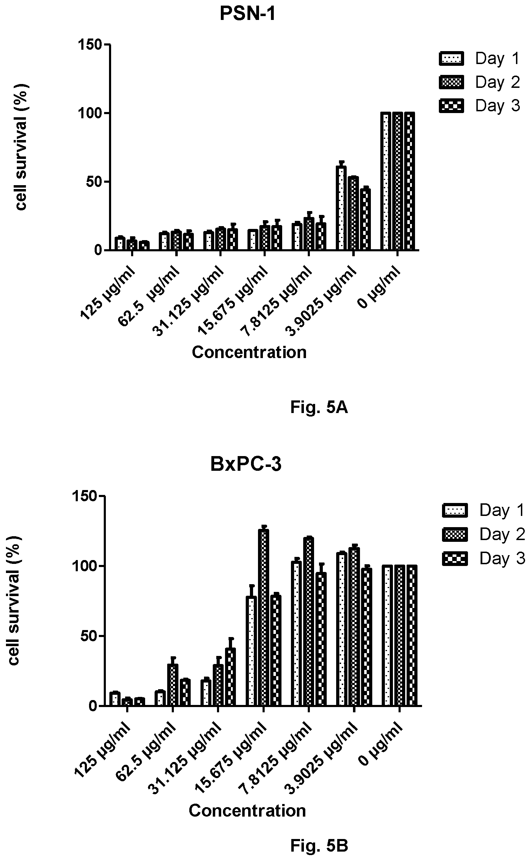

The perborate salts were found to have significant toxicity to cancer cell lines, with minimal toxicity to healthy cell lines (see Figures 1-9). For example, the perborate salts were found to be surprisingly effective at selectively killing cancer cells, preventing cancer cells from dividing and multiplying, moving and migrating to other tissues and organs, and preventing the attachment of cancer cells already present in the body to tissues and organs.

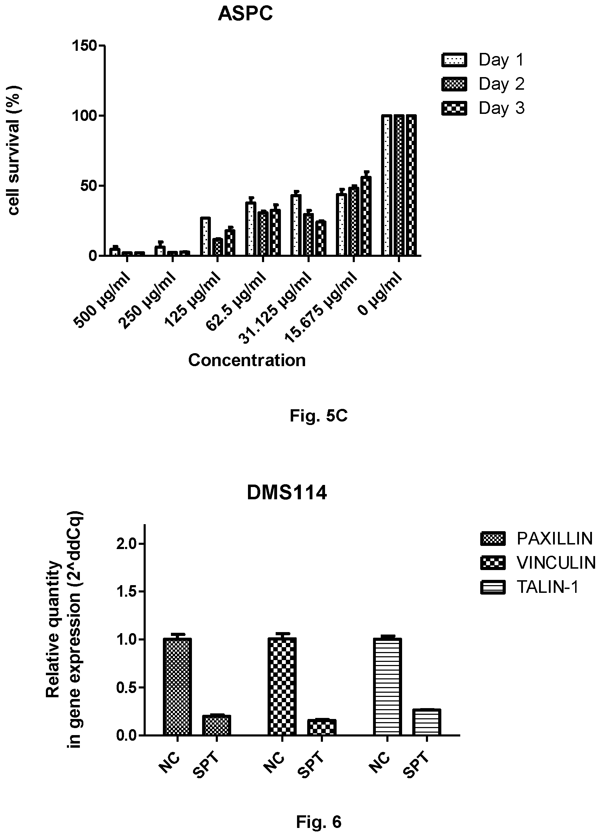

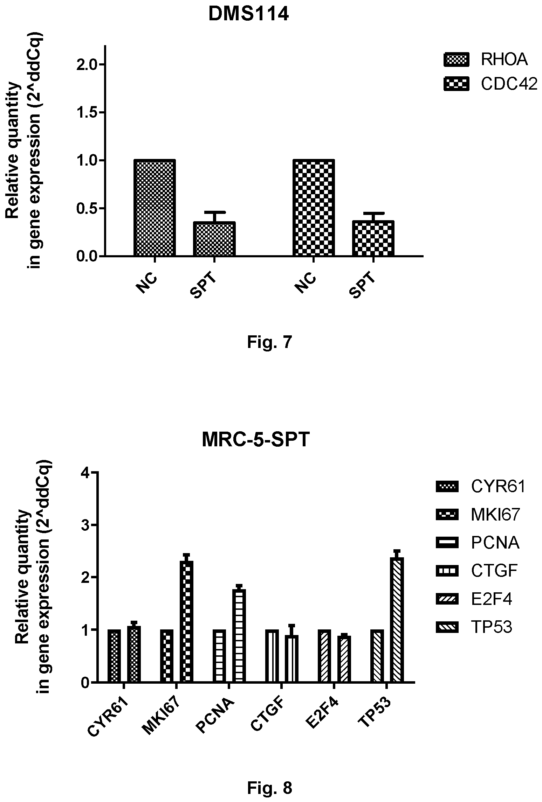

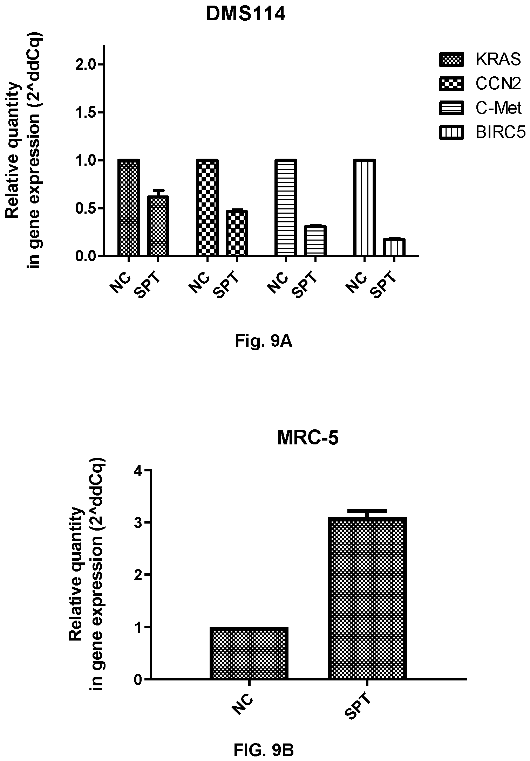

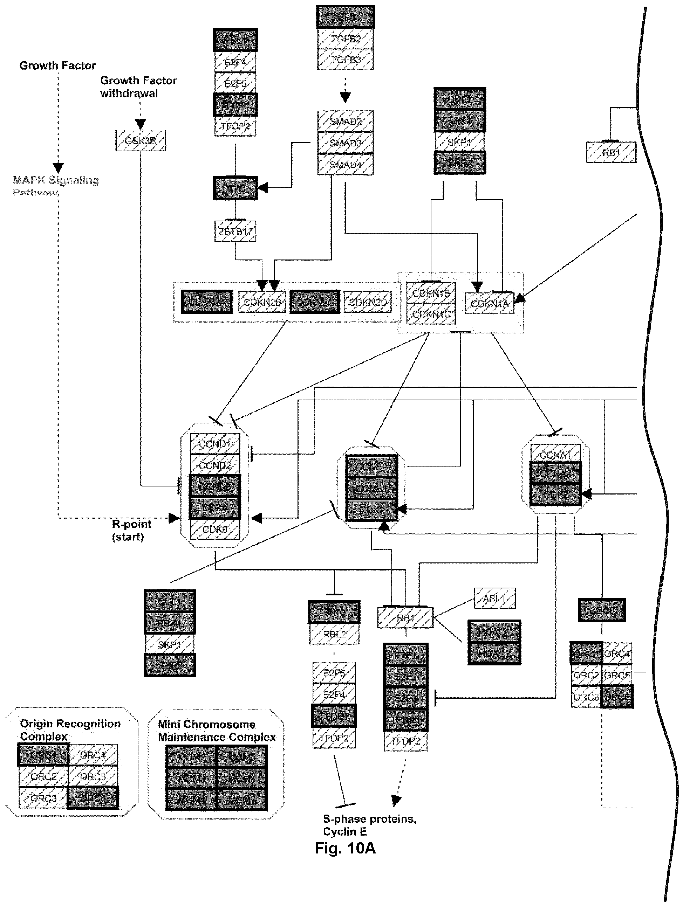

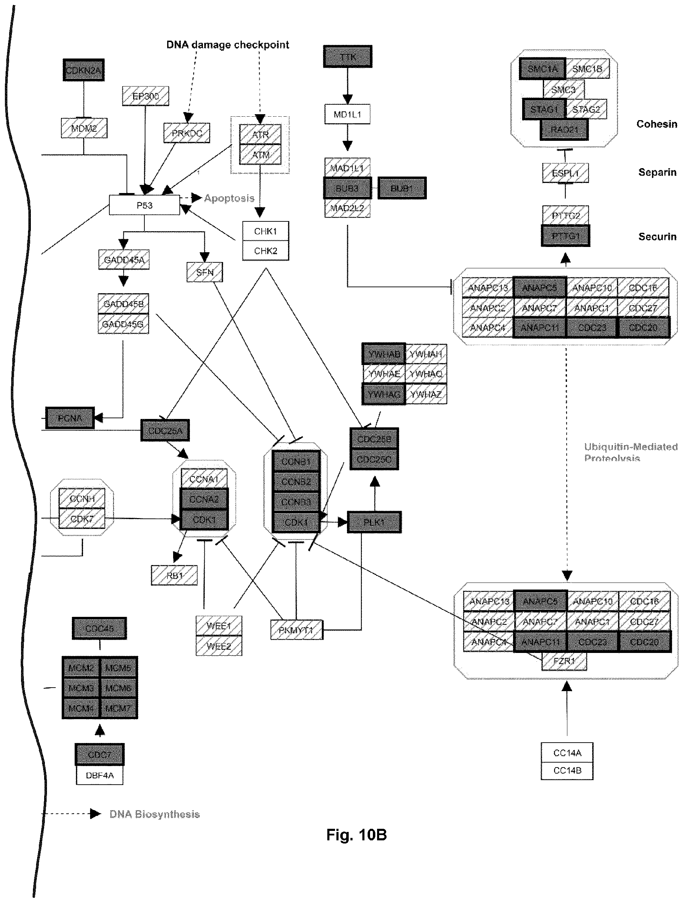

The following anti-cancer activities were observed, but are not exhaustive:
- Reducing expression of (blocking) focal adhesion proteins Paxillin, Vinculin, Talin-1, therefore preventing metastasis (see Figure 6);
- Reducing expression of Rho family proteins RhoA and CDC42, therefore preventing proliferation, metastasis and adhesion (see Figure 7);
- Reducing expression of the KRAS gene, an oncogene precursor that causes mutations in cancer cells and allows cancer cells to escape apoptosis (see Figure 9A);
- Reducing expression of the CCN2 gene, therefore preventing division, vascularization and migration of cancer cells (see Figure 9A);
- Reducing expression of survivin (BIRC5) and C-Met genes which would usually prevent cancer cells from escaping apoptosis (see Figure 9A); and
- Downregulation of cell signaling pathways including: cell cycle pathway, DNA replication pathway, EGF / EGFR signal pathway, focal adhesion-PI3K-Akt-mTOR signaling pathway, focal adhesion pathway, G protein signaling pathway, Hippo-Merlin signaling pathway, MAPK signaling pathway, actin cytoskeleton pathway, TGF-beta signaling pathway, Wnt signaling path (see Figures 10-20).

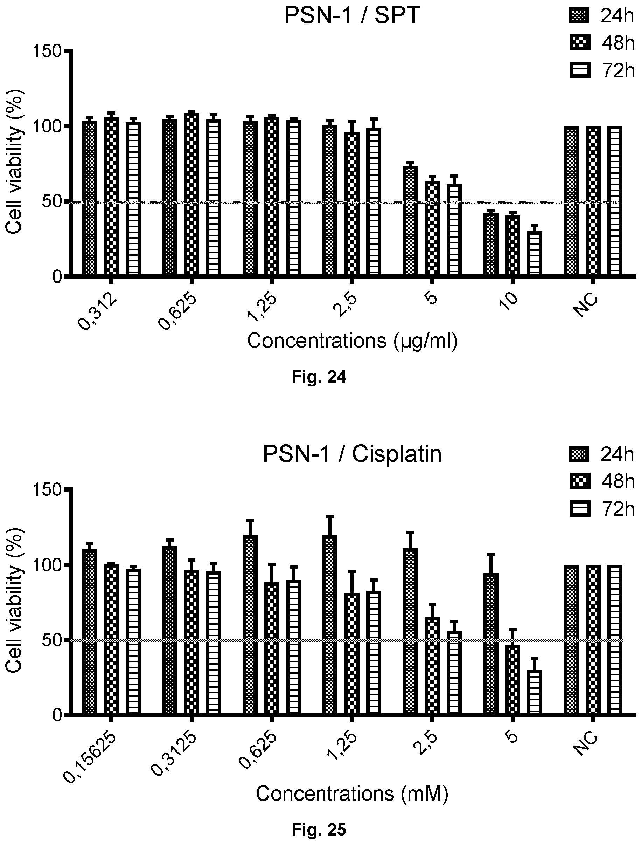

It was also found that administering a perborate salt in combination with a chemotherapy agent significantly reduced the concentration of the chemotherapy agent required to achieve an anti-cancer effect (see Figures 24-28).

The term "reducing gene expression", otherwise known as gene silencing, gene knockdown or gene suppression, will be readily understood by those in the art to mean any effect that renders a gene inactive, or less active. For example, a gene that is suppressed two-fold means that said gene has half of its usual level of activity. Methods for measuring gene activity will be known to those skilled in the art. For example, gene expression measurement may be achieved by quantifying levels of the gene product, which is often a protein (e.g. Western Blot, ELISA), or by measuring levels of mRNA or DNA (e.g. Northern Blot, Microarray). Interestingly, it was also found that the perborate salts increased the expression of survivin in healthy cells (see Figure 9B).

While existing cancer drugs generally produce a single effect (e.g. suppressing division), the use of perborate salts may produce multiple anti-cancer effects simultaneously, producing rapid and effective results, with no or minimal side effects compared to current treatments.

### Pharmaceutical compositions

The pharmaceutical compositions of the present invention may include a pharmaceutically acceptable carrier, diluent or adjuvant to achieve a composition usable as a dosage form. The pharmaceutical composition may be formulated for administration orally, intravenously, intraperitoneally, rectally, intramuscularly, or subcutaneously. In some embodiments, the pharmaceutical compositions are formulated for oral use.

The pharmaceutical compositions according to the invention can be in different forms, in particular in a form chosen from the group comprising tablets, capsules, pills, syrups, suspensions, solutions, powders, granules, emulsions, microspheres and injectable solutions and solid lipid nanoparticles, in particular solid lipid nanoparticles.

The pharmaceutical compositions of the present invention may be used alone or in combination with other biologically active ingredients. A pharmaceutical composition of the present invention, alone or in combination with other active ingredients, may be administered to a subject in a single dose or multiple doses over a period of time. In various embodiments, the perborate salts may be used as a pre-treatment or a co-treatment of a cancer in a subject. In some embodiments, the perborate salts may be formulated in a pharmaceutical composition in combination with a chemotherapy agent. In some embodiments, the perborate salts may be administered in combination with a chemotherapy agent. In some embodiments, the administration of the perborate salt with the chemotherapy agent is simultaneous. In some embodiments, the administration of the perborate salt with the chemotherapy agent is sequential.

### Dosages

The term "effective amount" of a perborate salt or a pharmaceutical composition comprising a perborate salt includes a therapeutically effect amount or a prophylactically effective amount. A "therapeutically effective amount" refers to an amount effective, at dosages and for periods of time necessary, to achieve the desired therapeutic result, such as decreased cancer proliferation, increased life span or increased life expectancy. A therapeutically effective amount may vary according to factors such as the disease state, age, sex and weight of the subject, and the ability of the perborate salt to elicit a desired response in the subject. Dosage regimens may be adjusted to provide the optimum therapeutic response. A therapeutically effective amount is also one in which any toxic or detrimental effects of the formulation are outweighed by the therapeutically beneficial effects. A "prophylactically effective amount" refers to an amount effective, at dosages and for periods of time necessary, to achieve the desired prophylactic result, such as the prevention, or the prevention of the progression, of a cancer. Typically, a prophylactic dose is used in subjects prior to or at an earlier state of disease. Methods of determining reduction or elimination of cancer or tumor cells are well known in the art, and need not be repeated herein.

It is to be noted that dosage values may vary with the severity of the condition to be alleviated. For any particular subject, specific dosage regimens may be adjusted over time according to the individual need and the professional judgment of the person administering or supervising the administration of the compositions. The amount of composition may vary according to factors such as the disease state, age, sex and weight of the subject. Dosage regimens may be adjusted to provide the optimum therapeutic response. For example, a single bolus may be administered, several divided doses may be administered over time or the dosage may be proportionally reduced or increased as indicated by the exigencies of the therapeutic situation. It may be advantageous to formulate compositions in dosage unit form for ease of administration and uniformity of dosage.

In some embodiments, the "effective amount" of perborate salt refers to a dosage of at least about 0.05 mg/kg/day. The dosage may be at least about 0.1 mg/kg/day. The dosage may be at least about 0.125 mg/kg/day. The dosage may be at least about 0.15 mg/kg/day. The dosage may be at least about 0.2 mg/kg/day. The dosage may be at least about 0.25 mg/kg/day. The dosage may be at least about 0.5 mg/kg/day. The dosage may be at least about 1 mg/kg/day. The dosage may be at least about 2 mg/kg/day. The dosage may be at least about 3 mg/kg/day. The dosage may be at least about 4 mg/kg/day. The dosage may be at least about 5 mg/kg/day. The dosage may be at least about 6 mg/kg/day. The dosage may be at least about 7 mg/kg/day. The dosage may be at least about 10 mg/kg/day. The dosage may be at least about 20 mg/kg/day. The dosage may be at least about 30 mg/kg/day. The dosage may be at least about 40 mg/kg/day. The dosage may be at least about 50 mg/kg/day. The dosage may be at least about 60 mg/kg/day. The dosage may be at least about 70 mg/kg/day. The dosage may be at least about 80 mg/kg/day. The dosage may be at least about 90 mg/kg/day. The dosage may be at least about 100 mg/kg/day.

The dosage may be between about 0.05 mg/kg/day and about 15 mg/kg/day. The dosage may be between about 0.1 mg/kg/day and about 10 mg/kg/day. The dosage may be between about 0.125 mg/kg/day and about 8 mg/kg/day. The dosage may be between about 0.05 mg/kg/day and about 7 mg/kg/day. The dosage may be between about 0.05 mg/kg/day and about 5 mg/kg/day. The dosage may be between about 10 mg/kg/day and about 20 mg/kg/day. The dosage may be between about 10 mg/kg/day and about 50 mg/kg/day. The dosage may be between about 20 mg/kg/day and about 50 mg/kg/day. The dosage may be between about 20 mg/kg/day and about 70 mg/kg/day. The dosage may be between about 10 mg/kg/day and about 120 mg/kg/day. The dosage may be between about 50 mg/kg/day and about 120 mg/kg/day. The dosage may be between about 50 mg/kg/day and about 70 mg/kg/day. The dosage may be between about 70 mg/kg/day and about 120 mg/kg/day.

The dosage may be up to about 0.25 mg/kg/day, up to about 0.5 mg/kg/day, up to about 1 mg/kg/day, up to about 2 mg/kg/day, up to about 4 mg/kg/day, up to about 5 mg/kg/day, up to about 6 mg/kg/day, up to about 7 mg/kg/day, up to about 8 mg/kg/day, up to about 10 mg/kg/day, or up to about 15 mg/kg/day. In some embodiments, the dosage of sodium perborate tetrahydrate may be up to about 50 mg/kg/day. In some embodiments, the dosage of sodium perborate tetrahydrate may be up to about 70 mg/kg/day. In some embodiments, the dosage of sodium perborate tetrahydrate may be up to 115 mg/kg/day.

In some embodiments, perborate salts as described herein may be used in combination with other treatment methods. For example, the perborate salts may be used as neoadjuvant (prior), adjunctive (during), and/or adjuvant (after) therapy in combination with other therapies known to one of ordinary skill in the art. As discussed above, the perborate salts may be administered in combination with a chemotherapy agent. In some embodiments, a non-toxic dosage of the perborate salt is used in a combination treatment, for example, up to about 115 mg/kg/day of sodium perborate tetrahydrate, up to about 70 mg/kg/day or up to about 15 mg/kg/day. In some embodiments, the effective dosage of the chemotherapy agent is at least 30% lower than a standard dosage of the chemotherapy agent. In some embodiments, the effective dosage of the chemotherapy agent is at least 40% lower than a standard dosage of the chemotherapy agent. In some embodiments, the effective dosage of the chemotherapy agent is at least 50% lower than a standard dosage of the chemotherapy agent. In some embodiments, the dosage of the chemotherapy agent is the EC50 dose or lower. In some embodiments, the dosage of the perborate salt is a dose that does not kill a cancer cell. Thus, the skilled person will appreciate that such dose of perborate salt that is used in a combination treatment will depend on the cancer being treated.

The perborate salts may be administered to a subject. As used herein, a "subject" may be a human, non-human primate, rat, mouse, cow, horse, pig, sheep, dog, cat, etc. The subject may be suspected of having or at risk for having a cancer. Diagnostic methods for cancers are known to those of ordinary skill in the art.

### Lung cancer

Lung cancer is divided into two subtypes: small cell lung cancer and non-small cell lung cancer. Approximately 85% of lung cancer cases are non-small cell lung cancers and 15% are small cell lung cancers. Small cell lung cancer cells are much more aggressive than the other type as they have the ability to divide rapidly, and they can easily metastatize to other tissues, especially the brain, bone and lymph, through the blood vessels (Nana-Sinkam & Powell, 2013). Surgical, radiotherapeutic and chemotherapeutic methods are generally used in the treatment of lung cancer. In cases of non-small cell lung cancer with a more mild prognosis than small cell lung cancer, tyrosine kinase inhibitors (such as axitinib and cediranib), small molecule inhibitors (such as cl-994 and cisplatin) and monoclonal antibodies (such as bevacizumab and figitumumab) are used for treatment (Lemjabbar-Alaoui et al., 2015). In small cell lung cancer, small molecule inhibitors such as cisplatin and etoposide are commonly used. However, despite these treatments, 98% of small cell lung cancer patients die within 5 years (Jackman & Johnson, 2005).

The present disclosure shows that perborate salts may be an effective treatment against various lung cancers and have minimal toxicity to healthy lung tissue.

### Pancreatic cancer

Pancreatic cancer is one of the most common cancer types and the mortality rate within 5 years is 95% (Jackman & Johnson, 2005). Pancreatic cancer, which has 2 subspecies: exocrine and endocrine, causes the death of 500 thousand people every year. Pancreatic cancer is the fastest spreading cancer in the body. It is known to metastasize to bone, brain, liver and lung tissues (Simon et al., 2018). In pancreatic cancer, surgery is performed if possible before chemotherapy treatment. The most commonly used chemotherapy drugs for pancreatic cancer are gemicitabin and 5-fluorouracil (Zhang & Yang, 2020).

The present disclosure shows that perborate salts may be an effective treatment against pancreatic cancer and have minimal toxicity to healthy tissue.

### Chordoma

Although a rare type of cancer, chordoma is an aggressive bone tumor that is thought to originate from ectopic notochord residues in the embryonic period encountered on the axial skeleton, with a high risk of recurrence and local metastasis. It is observed on the clivus and axial skeleton (Chugh et al., 2007). For chordomas, radical surgery is preferred as the main treatment method, since chordomas are resistant to chemotherapy and radiotherapy (Carrabba et al., 2008). The 5 year survival rate of chordoma is about 30-50% (Golden & Small, 2019).

The present disclosure shows that perborate salts may be an effective treatment against chordoma and have minimal toxicity to healthy tissue.

### Cell cycle pathway

The cell cycle pathway refers to the phases of cell division. In order for the cell to divide and multiply, this pathway must work effectively. It has been found that perborate salts can suppress many genes in this pathway and therefore prevent cancer cell division. Genes involved in the cell cycle pathway whose expression was found to decrease two-fold and above following treatment with a perborate salt include: ANAPC5, ANAPC11, CCNA2, CCNB1, CCNB2, CCNB3, CCND3, CCNE1, CCNE2, CDC20, CDC23, CDC25A, CDC25B, CDC25C, CDC45, CDC6, CDC7, CDK1, CDK2, CDK4, CDKN2A, CDKN2C, CUL1, E2F1, E2F2, E2F3, HDAC1, HDAC2, MCM2, MCM3, MCM4, MCM5, MCM6, MCM7, MYC, ORC1, ORC6, PCNA, PLK1, PTTG1, RAD21, RBL1, RBX1, BUB1, BUB3, SKP2, SMC1A, STAG1, TFDP1, TGFB1, TTK, YWHAB and YWHAG (see Figures 10A and 10B). On the other hand, it was found that gene expression of proliferation genes CYR61, MKI67, PCNA, CTGF, E2F4 and TP53 in healthy (MRC5) cells were not suppressed by perborate salts (see Figure 8).

### DNA replication pathway

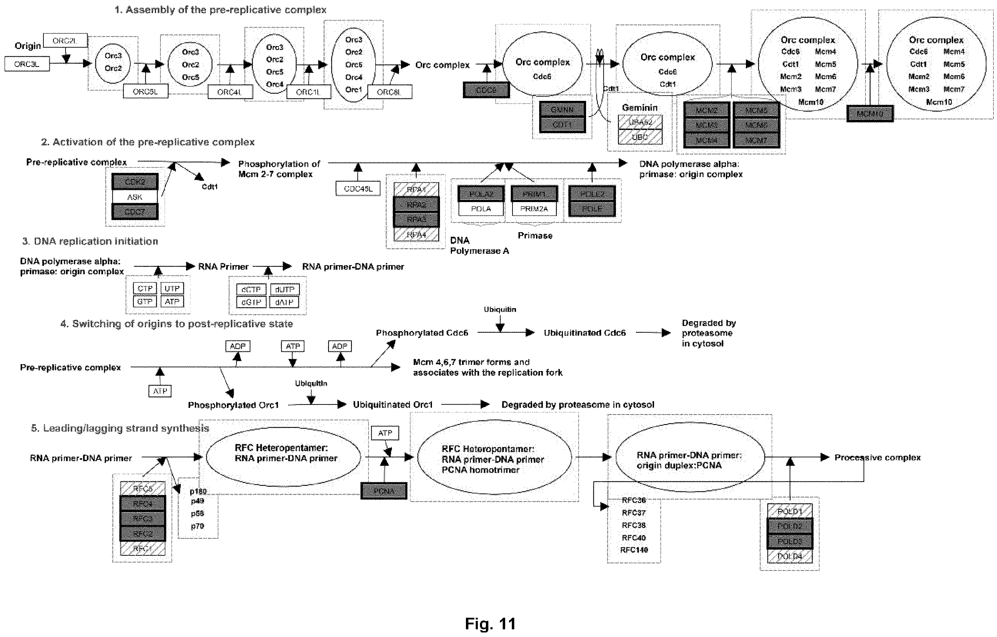

Cells must first copy their DNA in order to divide. Blocking the pathway for DNA replication prevents the cancer cell from dividing and multiplying. It has been found that perborate salts can prevent the division of cancer cells by lowering the expression levels of genes involved in the DNA replication pathway. Genes involved in the DNA replication pathway whose expression was found to decrease two-fold and above following treatment with a perborate salt include: CDC6, CDC7, CDK2, CDT1, GMNN, MCM10, MCM2, MCM3, MCM4, MCM5, MCM6, MCM7, PCNA, POLA2, POLD2, POLD3, POLE, POLE2, RFC2, RFC3, RFC4, RPA2, RPA3, and PRIM1 (see Figure 11).

### EGF/EGFR signal pathway

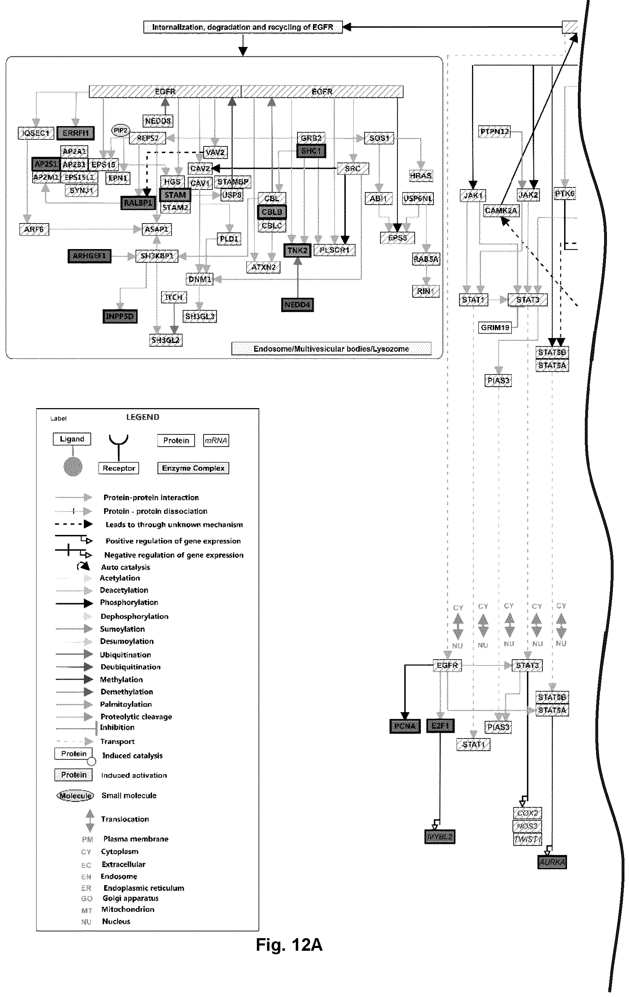

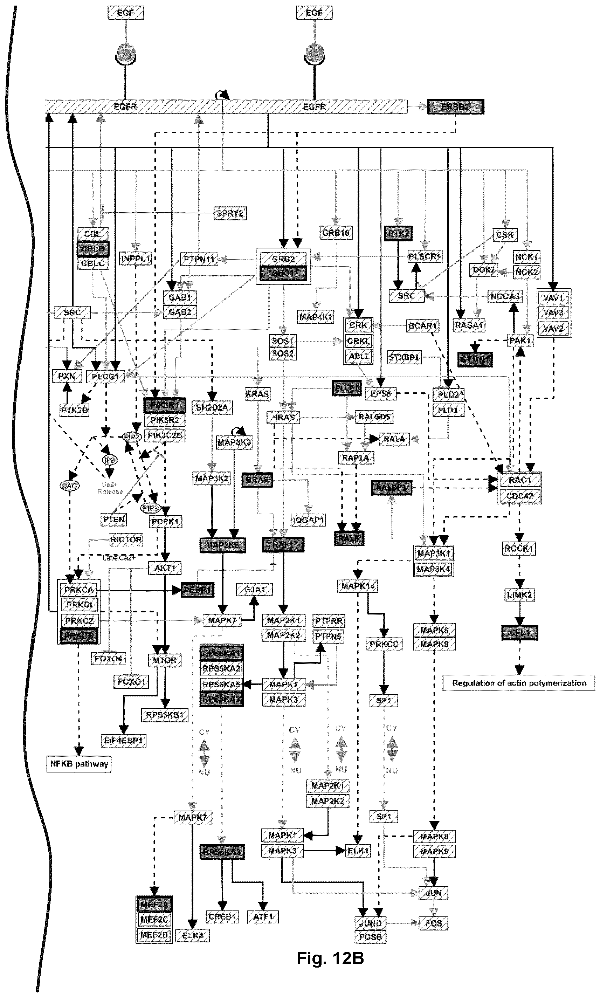

The epidermal growth factor receptor (EGFR) signaling pathway regulates growth, differentiation, migration, attachment and survival of cells. A disorder in this pathway prevents cancer cells from growing, differentiating, migrating, clinging and surviving. It has been found that perborate salts inhibit the function of the EGF pathway by suppressing the expression of genes in this pathway. Genes involved in the EGF/EGFR signaling pathway whose expression was found to decrease two-fold and above following treatment with a perborate salt include: AP2S1, ARHGEF1, AURKA, E2F1, INPP5D, MYBL2, NEDD4, PCNA, PEBP1, PIK3R1, PLCE1, PRKCB, RAF1, RALB, RALBP1, RPS6KA1, RPS6KA3, SHC1, and STMN1 (see Figures 12A and 12B).

The EGF/EGFR pathway is upregulated in many cancers, including bladder cancers, bone cancers, brain tumors, breast cancers, cervical cancers, colorectal cancers, endometrial cancers, gastric cancers (adenocarcinomas), liver cancers, lung cancers, lymphomas, oral cancers, ovarian cancers, pancreatic cancers, prostate cancers, renal cancers, skin cancers, thyroid cancers and leukemias. Thus, the perborate salts as disclosed herein, which inhibit the function of the EGF/EGFR pathway by suppressing expression of genes in this pathway, may be used to treat these cancers via downregulation of the EGF/EGFR pathway.

### Focal adhesion pathway

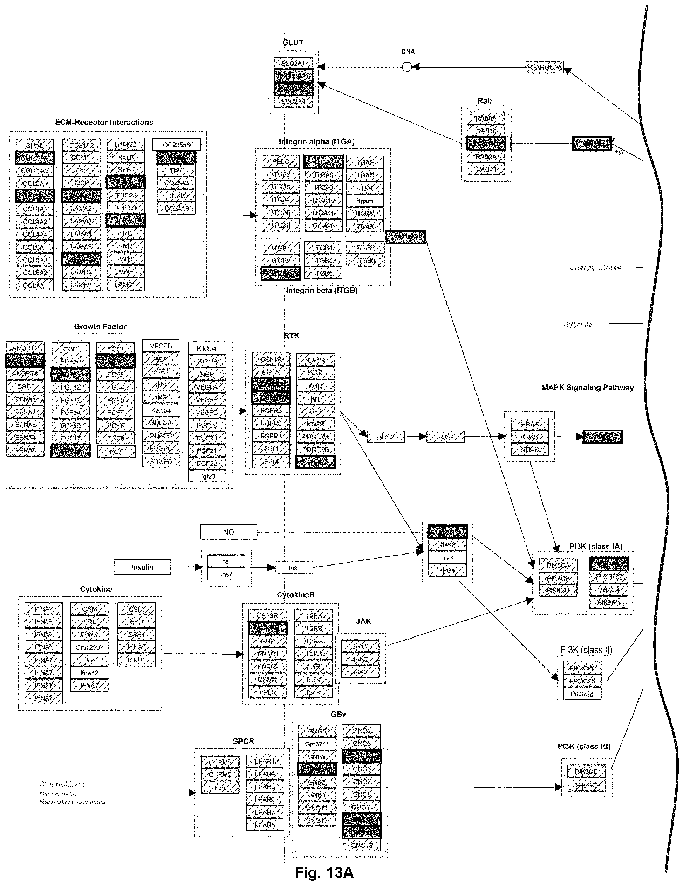

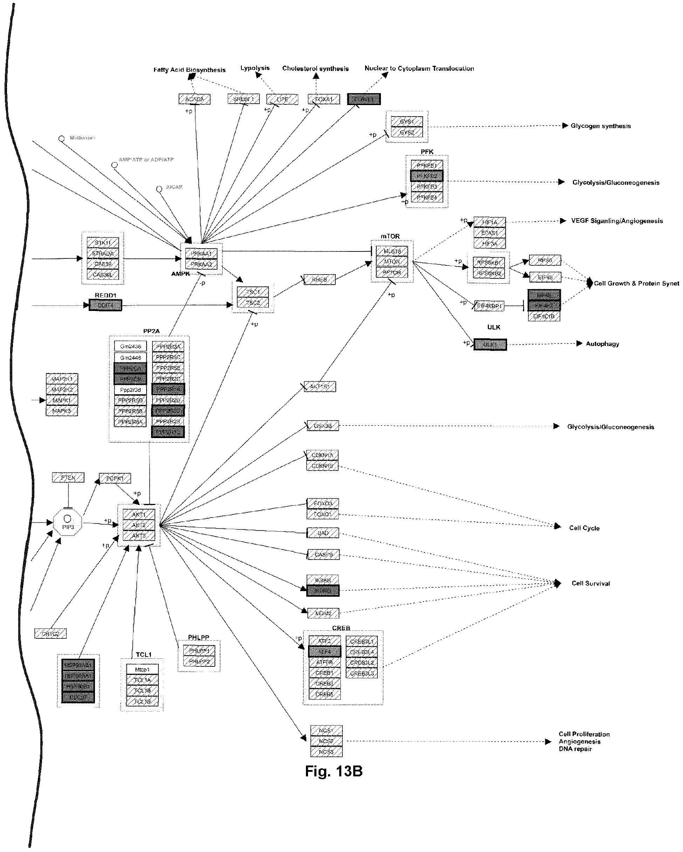

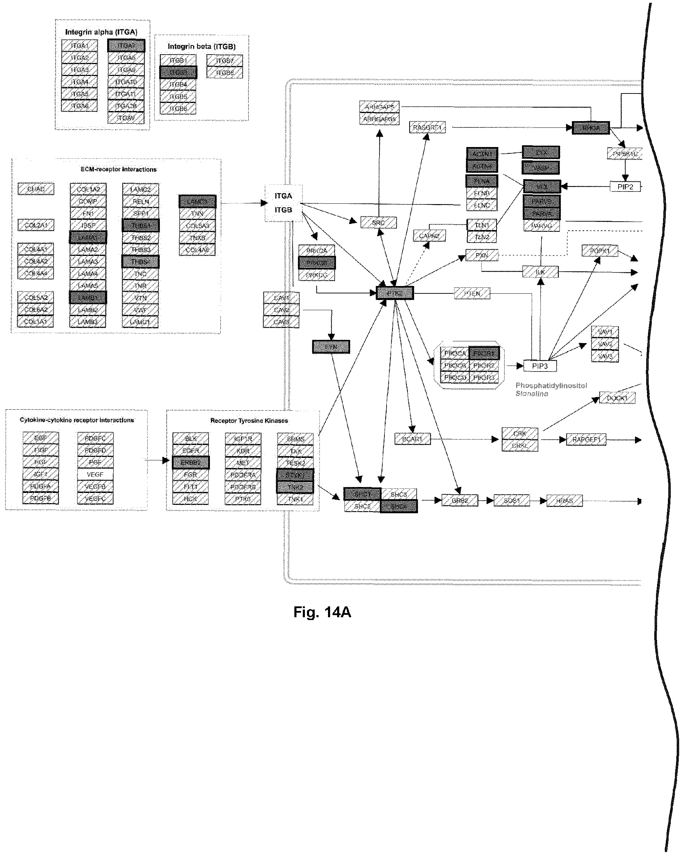

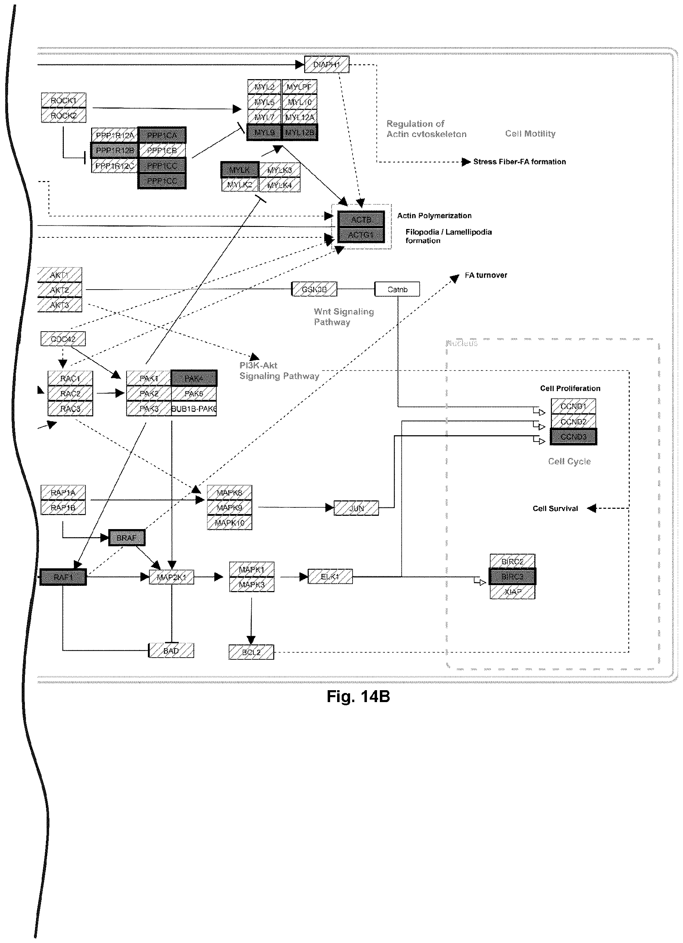

This pathway allows cells to move, e.g. allows cancer cells to metastasize by breaking off from one tissue and connecting to another. It has been found that perborate salts prevents cancer cells from moving and metastasizing by suppressing the expression of many genes in this pathway. Figures 13A and 13B are a pathway map showing genes involved in the focal adhesion pathway. Figures 14A and 14B show the focal adhesion pathway and its association with the PI3K-AKT-mTOR signaling pathway. Genes involved in the focal adhesion pathway whose expression was found to decrease two-fold and above following treatment with a perborate salt include: ANGPT2, CDC37, COL3A1, EIF4E, EIF4E2, ELAVL1, EPHA2, EPOR, FGF18, FGF2, FGFR1, GNB2, GNG4, GNG10, GNG12, HSP90AA1, HSP90AB1, HSP90B1, IKBKG, IRS1, ITGB3, LAMA1, LAMB1, LAMC3, PIK3R1, PPP2CA, PPP2CB, PPP2R1A, PPP2R1B, PPP2R3C, RAB11B, RAF1, SLC2A3, TBC1D1, THBS1 and ITGB3, LAMA1, LAMB1, THBS1, LAMC3, SHC1, SHC4, STYK1, PRKCB, ACTN1, ACTN4, FLNA, ZYX, VASP, VCL, PARVB, PARVA, RHOA, PIK3R1, PPP1CA, PPP1CC, MYL9, MYL12B, MYLK, ACTB, ACTG1, PAK4, CCND3, BIRC3, and RAF1 (see Figures 13A, 13B, 14A and 14B).

### G protein signaling pathway

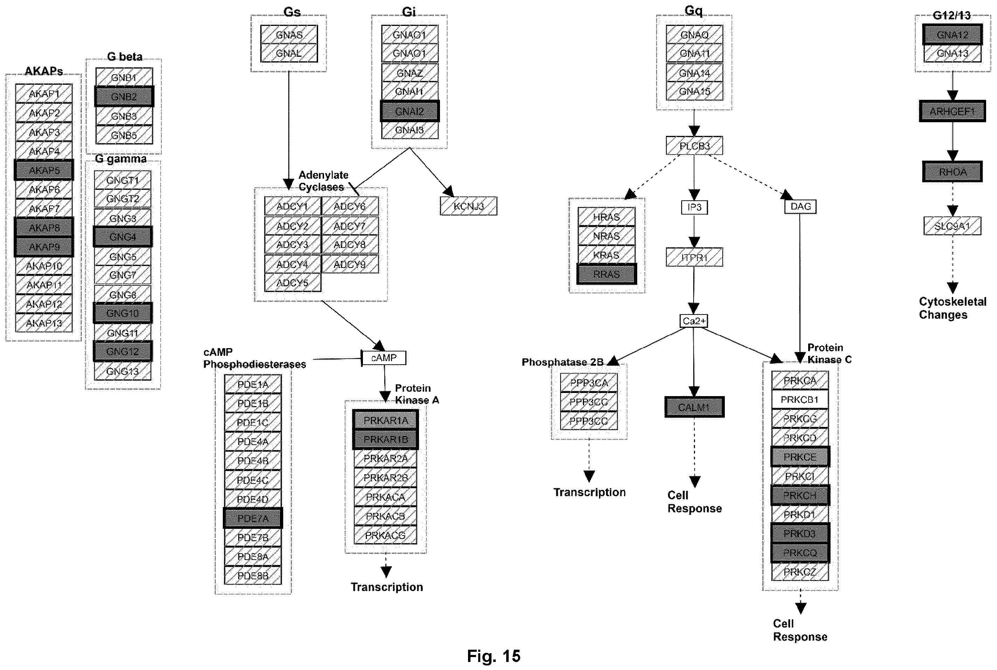

This pathway is a signal transmission pathway that enables the pathways inside the cell to work with an external stimulus. A disorder in this pathway will disrupt the signaling of cancer cells and inhibit its functions such as division, movement and growth. It has been found that perborate salts reduce the expression of many genes in this pathway. Genes involved in the G protein signaling pathway whose expression was found to decrease two-fold and above following treatment with a perborate salt include: AKAP5, AKAP8, AKAP9, GNB2, GNG4, GNG10, GNG12, GNAI2, PDE7A, PRKAR1B, RRAS, CALM1, GNA12, ARHGEF1, RHOA, PRKCH, PRKD3, and PRKCQ (see Figure 15).

### Hippo-Merlin Signaling dysregulation pathway

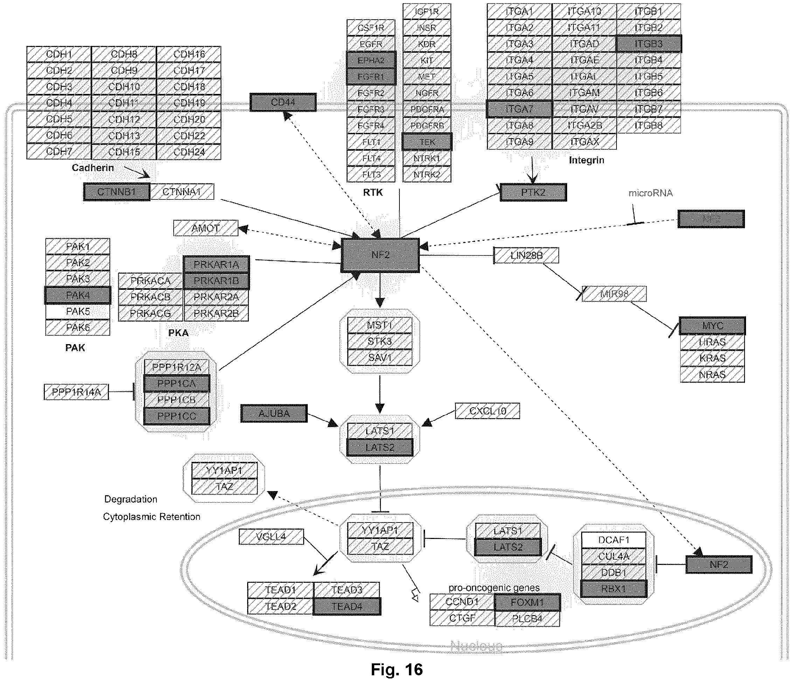

This pathway is related to the proliferation of cells and their apoptosis. A disorder in this pathway will prevent cancer cells from dividing and multiplying. It has been found that perborate salts disrupt the expression level of many genes in this pathway. Genes involved in the G protein signaling pathway whose expression was found to decrease two-fold and above following treatment with a perborate salt include: AJUBA, CD44, EPHA2, FGFR1, FOXM1, ITGB3, LATS2, MYC, NF2, PAK4, PPP1CA, PPP1CC, PRKAR1B, RBX1 and TEAD4 (see Figure 16).

### MAPK signaling pathway

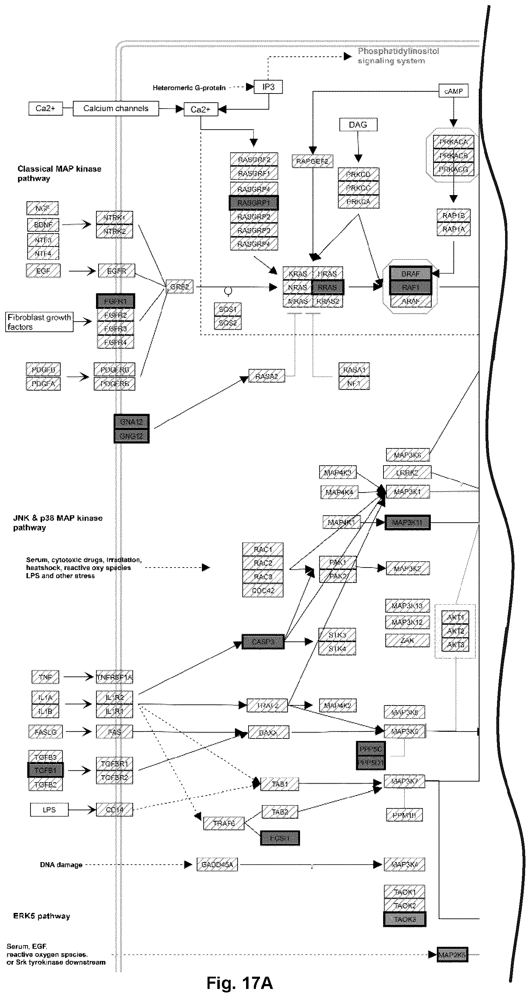

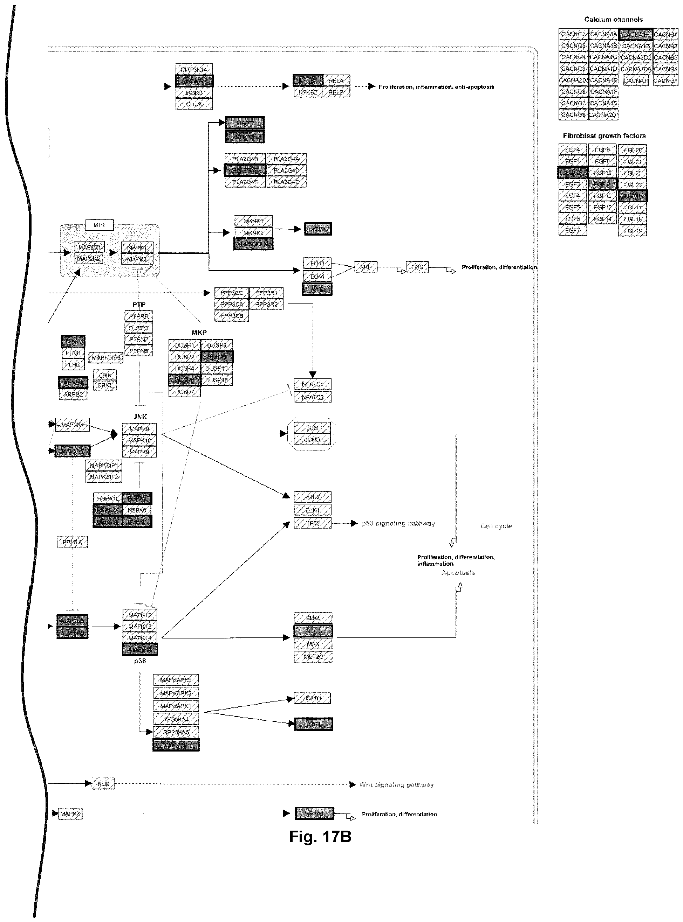

This pathway provides communication between the stimulator that binds to a receptor outside the cell and the cell nucleus. A defect in this pathway negatively affects many functions, primarily cell division and cell cycle. It has been found that perborate salts reduce the expression of many genes in this pathway. Genes involved in the MAPK signaling pathway whose expression was found to decrease two-fold and above following treatment with a perborate salt include: ARRB1, CASP3, CDC25B, DUSP6, DUSP9, ECSIT, FGF18, FGF2, FGFR1, FLNA, GNA12, HSPA1A, HSPA1B, HSPA2, HSPA8, IKBKG, MAP2K3, MAP2K6, MAP2K7, MAP3K11, MAPK11, MYC, NFKB1, PLA2G4E, PPP5C, PPP5D1, RAF1, RASGRP1, RPS6KA3, RRAS, STMN1, and TGFB1 (see Figure 17A and 17B).

### Actin-cytoskeleton pathway

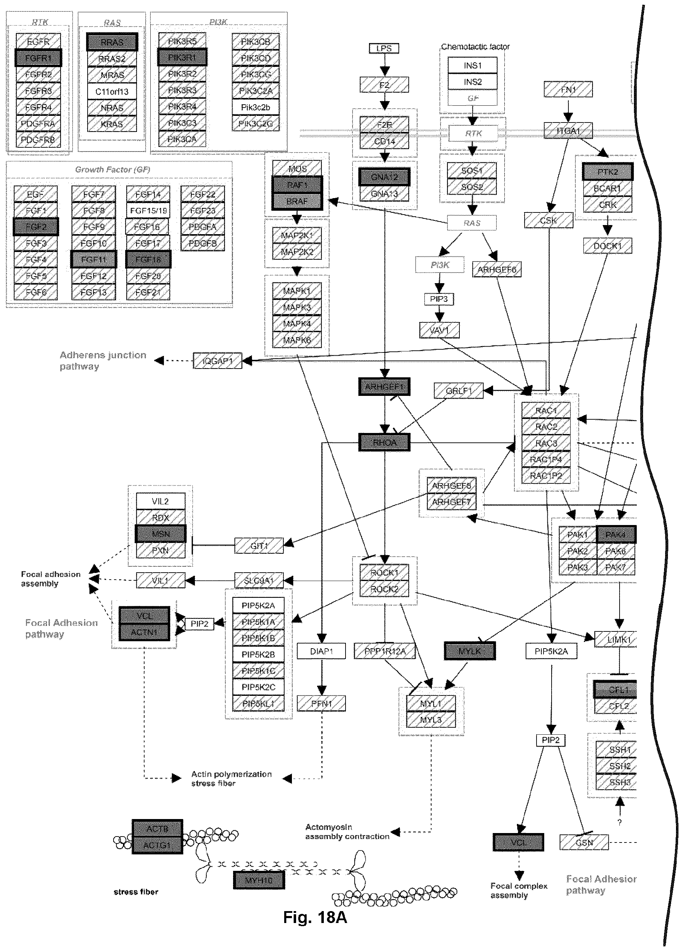

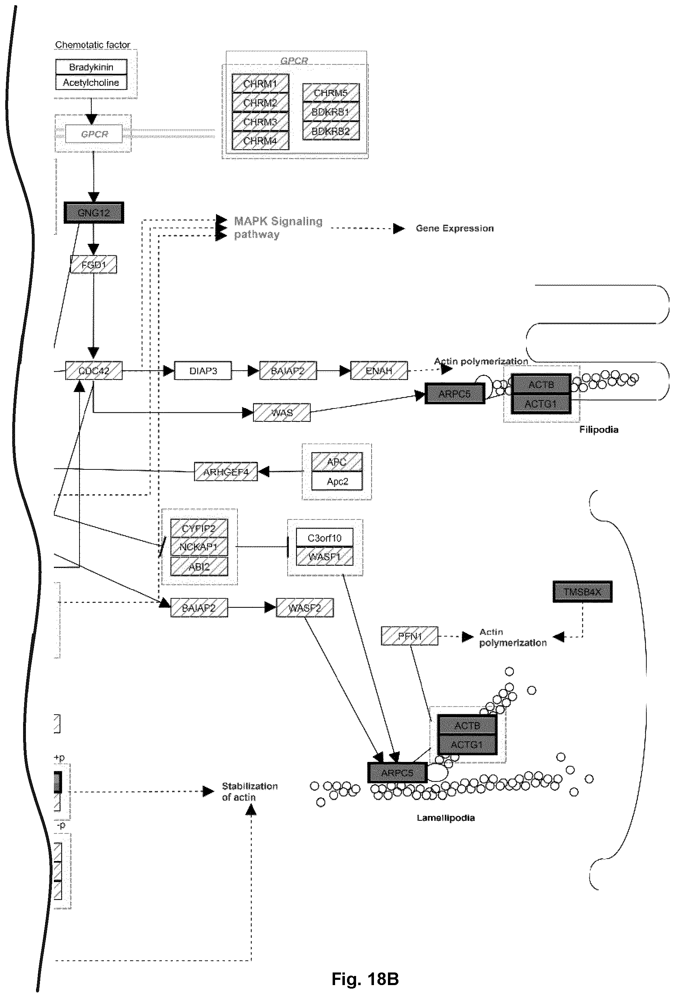

This pathway is another basic pathway that allows cells to move. A disorder in this pathway will prevent a cancer cell from moving and as a result, prevent metastasis and spreading. It has been found that perborate salts reduce the expression of many genes in this pathway. Genes involved in the actin-cytoskeleton pathway whose expression was found to decrease two-fold and above following treatment with a perborate salt include: ACTB, ACTG1, ACTN1, ARHGEF1, ARPC5, FGF18, FGF2, FGFR1, GNG12, MSN, MYH10, MYLK, PAK4, PIK3R1, RAF1, RHOA, RRAS, TMSB4X, and VCL (see Figure 18A and 18B).

### TGF Beta signaling pathway

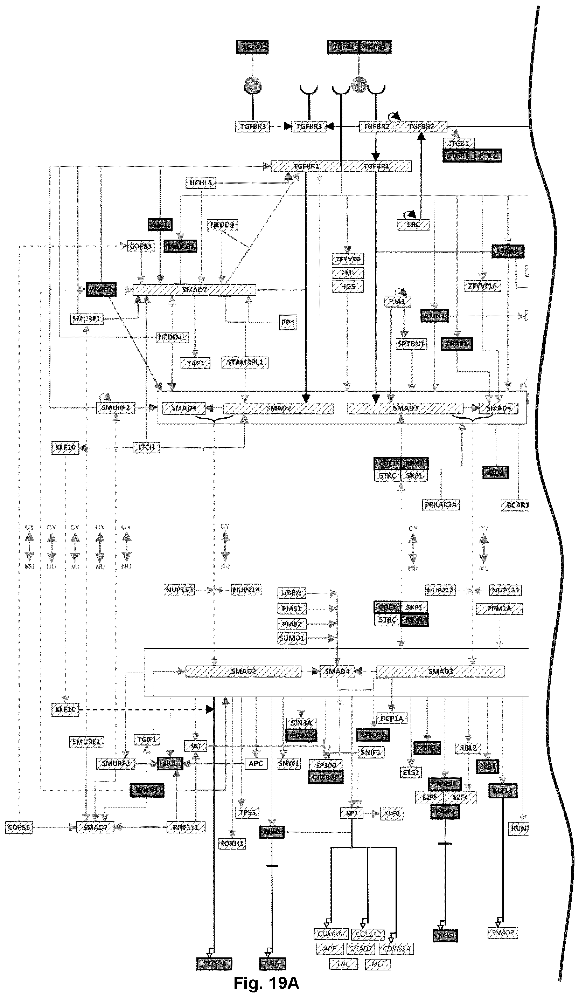

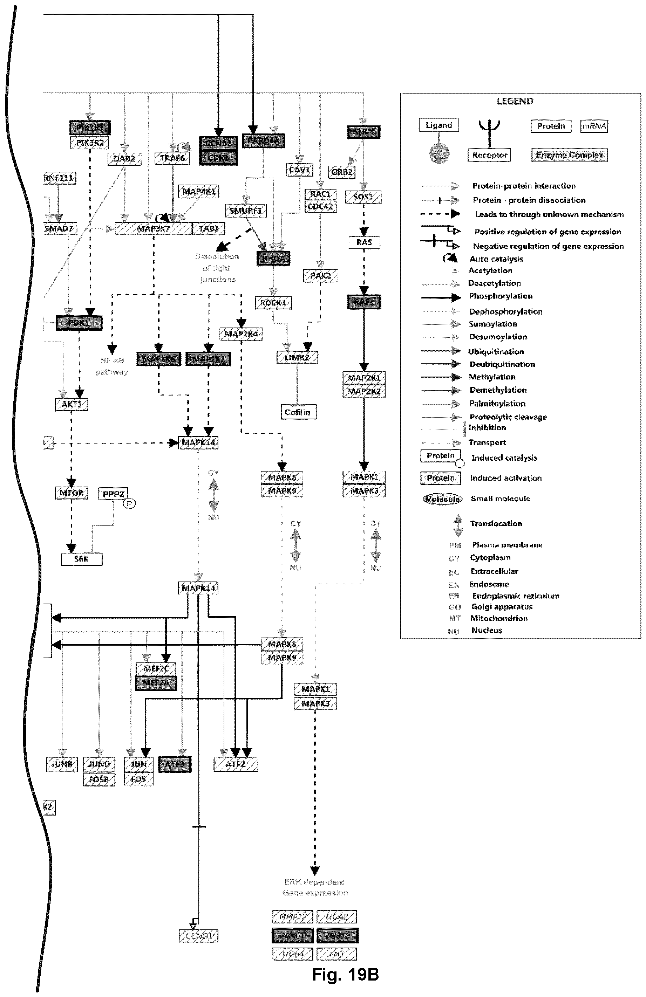

This pathway is one of the main pathways that controls the differentiation and growth of cells. A disorder in this pathway will prevent the growth and differentiation of cancer cells. It has been found that perborate salts reduce the expression level of many genes in this pathway. Genes involved in the TGF Beta signaling pathway whose expression was found to decrease two-fold and above following treatment with a perborate salt include: CCNB2, CDK1, CITED1, CUL1, EID2, FOXP3, HDAC1, ITGB3, MAP2K6, MMP1, MYC, PARD6A, PIK3R1, RAF1, RBL1, RBX1, RHOA, SHC1, SIK1, TERT, TFDP1, TGFB1, TGFB1I1, THBS1, TRAP1, WWP1, and ZEB2 (see Figure 19A and 19B).

### WNG signaling pathway

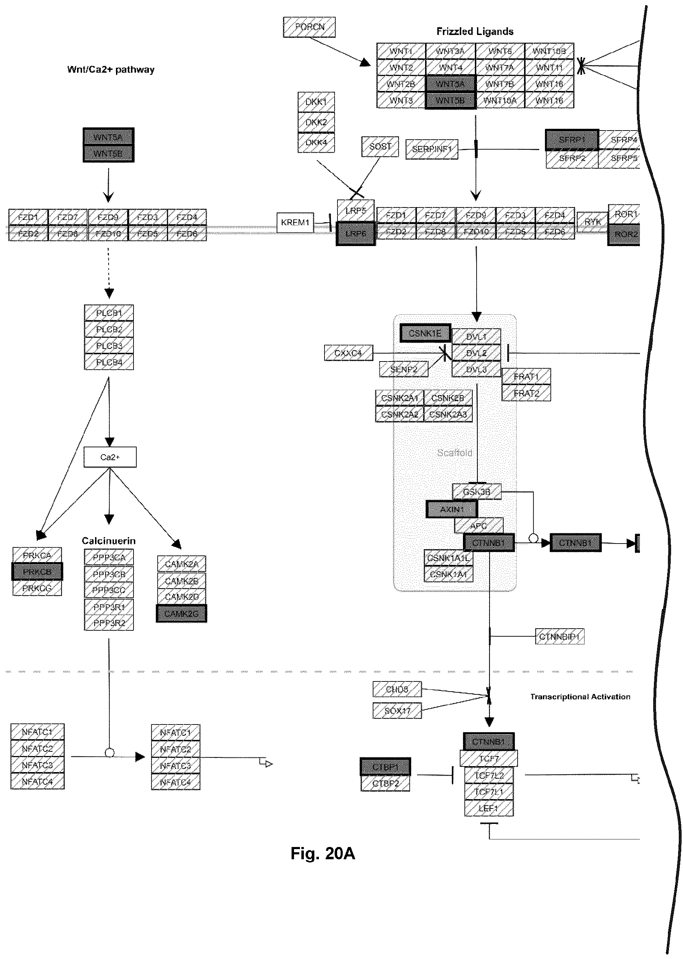

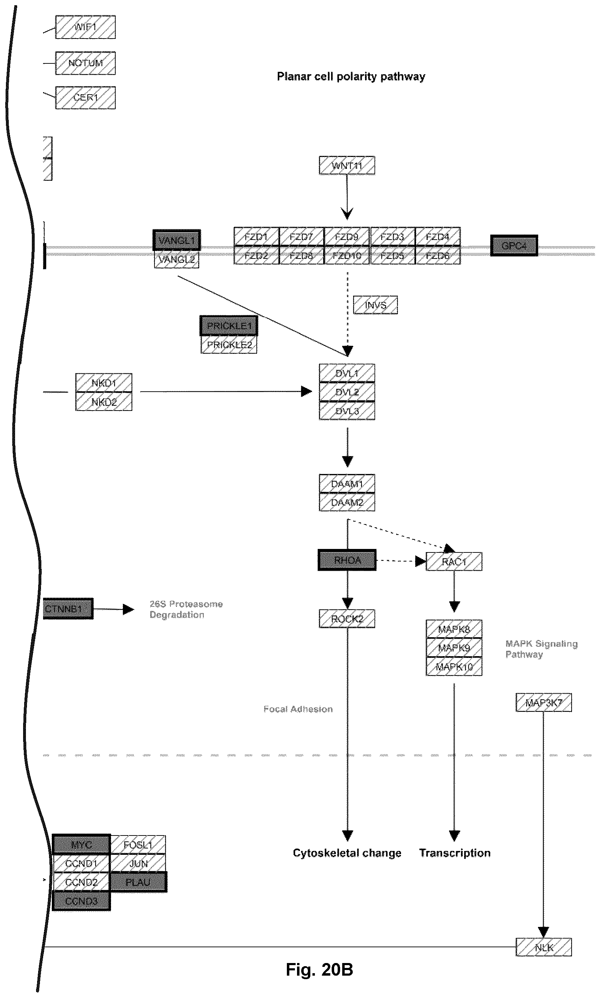

This pathway controls the intracellular signal pathways that start with a stimulus that binds to the cell receptors. It is one of the pathways that control the destiny, movement and migration of the cell. A disorder in this pathway prevents the cell from continuing its life and prevents its division and migration. It has been found that perborate salts reduce the expression level of many genes in this pathway. Genes involved in the WNG signaling pathway whose expression was found to decrease two-fold and above following treatment with a perborate salt include: CAMK2G, CCND3, CTBP1, CTNNB1, GPC4, LRP6, MYC, PLAU, PRICKLE1, PRKCB, RHOA, ROR2, SFRP1, VANGL1, WNT5A, and WNT5B (see Figure 20A and 20B).

### EXAMPLES

These examples illustrate various aspects of the disclosure, evidencing a variety of methods for reducing gene expression of one or more genes in a cancer or a tumor cell by contacting the cell with an effective amount of a perborate salt. Selected examples are illustrative of advantages that may be obtained compared to alternative methods, and these advantages are accordingly illustrative of particular embodiments.

As used herein, the term "about" refers to an approximately +/-10% variation from a given value. It is to be understood that such a variation is always included in any given value provided herein, whether or not it is specifically referred to.

### Example 1 - Flow cytometry (Annexin V)

Cell death pathways were analysed using the Annexin V method, performed by flow cytometry. In this method, Annexin V dye is used to detect cells entering the apoptosis death pathway (Sigma-Aldrich Cat No: 11858777001), while PI (Propidium Iodide) dye is used to detect cells entering the necrosis death pathway (Sigma-Aldrich Cat No: 11858777001).

### Method

Cells were grown in complete RPMI media (completed with 10% Fetal Bovine Serum and 1% penicillin streptomycin amphotericin) in a humidified CO₂ incubator (80% humidity, 5% CO₂ and 37 °C). At 70-80% confluency, cells were harvested using Trypsin/EDTA solution. After 3 days of sodium perborate tetrahydrate treatment (15 µg/mL), the cells were harvested and divided into 4 equal tubes. The first tube did not contain any dye and was used as a negative control. Only PI dye was placed in the second tube and necrotic cells were detected. Annexin V dye was placed in the third tube and apoptotic cells were detected. In the fourth tube, two dyes were put together. The result were analyzed and are shown in Figures 1 and 2. Figure 1 shows that in the control, ~80% of DMS114 cells were alive after 3 days, with ~15% of cells in early apoptosis and ~5% of cells in late apoptosis, whereas in the cells treated with sodium perborate tetrahydrate, less than 5% of cells were alive after 3 days, with ~80% in early apoptosis and ~10% in late apoptosis. On the other hand, over 90% of MRC5 cells were alive in the control as well as following treatment with sodium perborate tetrahydrate, with only a small percentage (<5%) of treated cells in necrosis. Sodium perborate tetrahydrate was shown to effectively kill small cell lung cancer cells (DMS114), while giving healthy cells (MRC5) approximately 5 times less damage than cancer cells.

### Example 2 - Cytotoxicity assay

An MTS reduction (MTS (3-(4,5-dimethylthiazol-2-yl)-5-(3-carboxymethoxyphenyl)-2-(4-sulfophenyl)-2H-tetrazolium) assay) was conducted to analyze the cytotoxic effect of sodium perborate tetrahydrate on lung, chordoma and pancreatic cells and normal cells. In this method, cell viability is determined via the activity of mitochondrial dehydrogenase enzyme, which reduces the tetrazolium dye MTS to its insoluble formazan (absorbance maximum of 490 nm in PBS).

### Method

Cells were counted and seeded in 96-well culture dishes. BxPC3 and PSN-1 cells were contacted with sodium perborate tetrahydrate at concentrations of 125 µg/mL, 62.5 µg/mL, 31.25 µg/mL, 15.625 µg/mL, 7.8125 µg/mL and 3.90615 µg/mL. DMS114 cells were contacted with sodium perborate tetrahydrate at concentrations of 250 µg/mL, 125 µg/mL, 62.5 µg/mL, 31.25 µg/mL, 15.625 µg/mL, 7.8125 µg/mL and 3.90615 µg/mL. ASPC and MRC5 cells were contacted with sodium perborate tetrahydrate at concentrations of 500 µg/mL, 250 µg/mL, 125 µg/mL, 62.5 µg/mL, 31.25 µg/mL, 15.625 µg/mL. CH22, JHC7, UM-Chor1, MUG-Chor1, UCH1 and NPH cells were contacted with sodium perborate tetrahydrate at concentrations of 500 µg/mL, 250 µg/mL and 125 µg/mL. The positive control comprised 20% Dimethyl Sulfoxide and the negative control comprised complete growth media. Seeding densities were as follows: DMS114: 20.000 cells/well; MRC5: 5.000 cells/well; CH22: 2.000 cells/well; JHC7, UM-Chor1, MUG-Chor1, UCH1 and NPH: 5.000 cells/well; PSN1: 10.000 cells/well; BxPC3 and ASPC: 7.500 cells/well. The effect of sodium perborate tetrahydrate on cell viability was measured as follows. The cell culture medium was discarded and 100 µl of MTS solution (10% glucose solution and 1% MTS-PMS solution in PBS) was added into each well. After 1 hour incubation in a humidified incubator, an absorbance reading was taken to determine mitochondrial dehydrogenase activity and cell survival was calculated relative to the negative control.

The results show that sodium perborate tetrahydrate was effective in decreasing cell survival of lung cancer, pancreatic and chordoma cells lines, with minimal impact on the cell survival of normal cells lines (see Figures 3A and 3B, Figures 4A-4F and Figures 5A-5C).

### Example 3 - Video Analysis of Cell Movement and Division

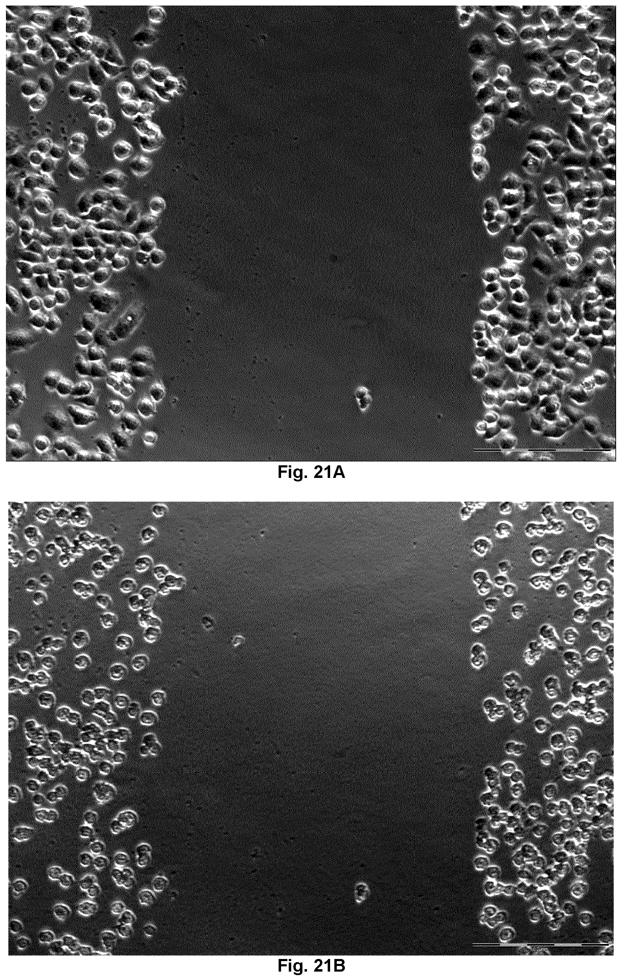

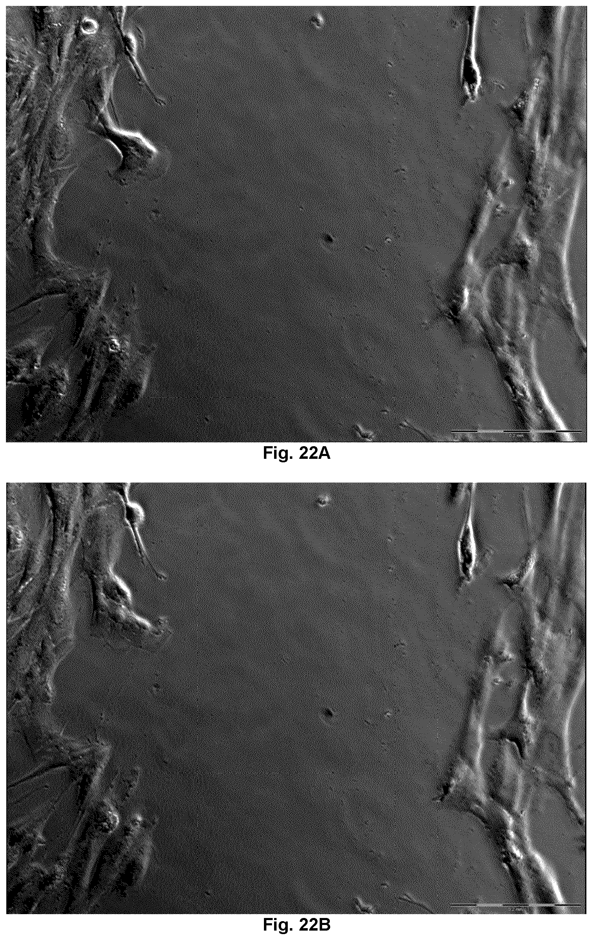

The DMS114 and MRC5 cells from Example 2 were analyzed during the 3 day treatment period with sodium perborate tetrahydrate. Roughly 20 photos/hour were taken of the cells over the 72 hour (3 day) period. The resulting 1500 were merged to create a video which was used to observe the effect of sodium perborate on movement and division of DMS114 and MRC5 cells. The video analysis showed that the maximum effect on cell survival of DMS114 cells was achieved after 18 hours and that movement of close to 100% of DMS114 cells was stopped. The video analysis also showed that sodium perborate tetrahydrate had the effect of slowing cell division and movement of healthy cells to some extent, but that the effects diminished once the sodium perborate tetrahydrate moved away from the cellular environment. Figures 21A and 21B show DMS114 cells at the start and end of the 72 hour treatment period. Figures 22A and 22B show MRC5 cells at the start and end of the 72 hour treatment period.

### Example 4 - Real Time Polymerase Chain Reaction (RT-PCR)

Changes in the expression level of the cells treated with sodium perborate tetrahydrate for 3 days were carried out using the polymerase chain reaction method. Primers used were created with Primer BLAST software on NCBI (National Center for Biotechnology). After sodium perborate tetrahydrate treatment, the total RNAs of the cells were isolated (Macherey-Nagel Cat No: 740955250) and cDNA synthesis was made from these RNAs (QIAGEN Cat No: 205313). Synthesized cDNAs were mixed with SYBR Green mixture (Biorad Cat No: 1725122) and required primers to a final volume of 10 µl and expression levels of genes were analyzed using a BIO-RAD^{™} PCR device. The results are shown in Figures 6-9.

### Example 5 - Microarray experiment

Cells were treated with sodium perborate tetrahydrate for 3 days and changes in the levels of gene expression were determined by Microarray. Affected genes and related pathways are shown in Figures 10-20. Genes where expression was reduced two-fold or more compared to the control are shown in grey shaded boxes on each pathway map. Genes where expression was reduced less than two-fold are shown in boxes with diagonal lines.

### Example 6 - Toxicology in rats

Rats were treated orally with varying dosages of sodium perborate tetrahydrate ranging from 0.125 mg/kg/day to 8 mg/kg/day. After 32 days, the rats were sacrificed and their organs were examined under a microscope. The histopathology results showed no significant organ damage (see Tables 1 and 2 below).

**Table 1: Tissues submitted for histology and numbers of rats examined**

| **Group#** | **1** | | **2** | | **3** | | **4** | | **5** | | **6** | | **7** | | **8** | |
|---|---|---|---|---|---|---|---|---|---|---|---|---|---|---|---|---|
| **Treatment** | Control | | Sodium Perborate Tetrahydrate | | Sodium Perborate Tetrahydrate | | Sodium Perborate Tetrahydrate | | Sodium Perborate Tetrahydrate | | Sodium Perborate Tetrahydrate | | Sodium Perborate Tetrahydrate | | Sodium Perborate Tetrahydrate | |
| **Treatment Dose** | 0 | | 0.125 mg/kg/day | | 0.25 mg/kg/day | | 0.5 mg/kg/day | | 1 mg/kg/day | | 2 mg/kg/day | | 4 mg/kg/day | | 8 mg/kg/day | |
| **Time of Sacrifice (in days)** | 32 | | 32 | | 32 | | 32 | | 32 | | 32 | | 32 | | 32 | |
| **#Rats/Group** | 10 (5 M, 5 F) | | 10 (5 M, 5 F) | | 10 (5 M, 5 F) | | 10 (5 M, 5 F) | | 10 (5 M, 5 F) | | 10 (5 M, 5 F) | | 10 (5 M, 5 F) | | 10 (5 M, 5 F) | |

| **Tissue** | No. Exam | % | No. Exam | % | No. Exam | % | No. Exam | % | No. Exam | % | No. Exam | % | No. Exam | % | No. Exam | % |
|---|---|---|---|---|---|---|---|---|---|---|---|---|---|---|---|---|
| **Lung** | 10/10 | 100 | 10/10 | 100 | 10/10 | 100 | 10/10 | 100 | 10/10 | 100 | 10/10 | 100 | 10/10 | 100 | 10/10 | 100 |
| **Liver** | 10/10 | 100 | 10/10 | 100 | 10/10 | 100 | 10/10 | 100 | 10/10 | 100 | 10/10 | 100 | 10/10 | 100 | 10/10 | 100 |
| **Kidney** | 10/10 | 100 | 10/10 | 100 | 10/10 | 100 | 10/10 | 100 | 10/10 | 100 | 10/10 | 100 | 10/10 | 100 | 10/10 | 100 |
| **Spleen** | 10/10 | 100 | 10/10 | 100 | 10/10 | 100 | 10/10 | 100 | 10/10 | 100 | 10/10 | 100 | 10/10 | 100 | 10/10 | 100 |
| **Heart** | 10/10 | 100 | 10/10 | 100 | 10/10 | 100 | 10/10 | 100 | 10/10 | 100 | 10/10 | 100 | 10/10 | 100 | 10/10 | 100 |
| **Pancreas** | 10/10 | 100 | 10/10 | 100 | 10/10 | 100 | 10/10 | 100 | 10/10 | 100 | 10/10 | 100 | 10/10 | 100 | 10/10 | 100 |
| **Intestine, Large** | 10/10 | 100 | 10/10 | 100 | 10/10 | 100 | 10/10 | 100 | 10/10 | 100 | 10/10 | 100 | 10/10 | 100 | 10/10 | 100 |
| **Intestine, Small** | 10/10 | 100 | 10/10 | 100 | 10/10 | 100 | 10/10 | 100 | 10/10 | 100 | 10/10 | 100 | 10/10 | 100 | 10/10 | 100 |
| **Stomach** | 10/10 | 100 | 10/10 | 100 | 10/10 | 100 | 10/10 | 100 | 10/10 | 100 | 10/10 | 100 | 10/10 | 100 | 10/10 | 100 |
| **Testes** | 5/10 | 50 | 5/10 | 50 | 5/10 | 50 | 5/10 | 50 | 5/10 | 50 | 5/10 | 50 | 5/10 | 50 | 5/10 | 50 |
| **Skeletal Muscle** | 10/10 | 100 | 10/10 | 100 | 10/10 | 100 | 10/10 | 100 | 10/10 | 100 | 10/10 | 100 | 10/10 | 100 | 10/10 | 100 |
| **Skin** | 10/10 | 100 | 10/10 | 100 | 10/10 | 100 | 10/10 | 100 | 10/10 | 100 | 10/10 | 100 | 10/10 | 100 | 10/10 | 100 |
| **Uterus** | 5/10 | 50 | 5/10 | 50 | 5/10 | 50 | 5/10 | 50 | 5/10 | 50 | 5/10 | 50 | 5/10 | 50 | 5/10 | 50 |
| **Tongue** | 10/10 | 100 | 10/10 | 100 | 10/10 | 100 | 10/10 | 100 | 10/10 | 100 | 10/10 | 100 | 10/10 | 100 | 10/10 | 100 |
| **Esophagus** | 10/10 | 100 | 10/10 | 100 | 10/10 | 100 | 10/10 | 100 | 10/10 | 100 | 10/10 | 100 | 10/10 | 100 | 10/10 | 100 |

**Table 2: Histopathology results**

| Group# | 1 | | 2 | | 3 | | 4 | | 5 | | 6 | | 7 | | 8 | |
|---|---|---|---|---|---|---|---|---|---|---|---|---|---|---|---|---|
| Treatment | Control | | Sodium Perborate Tetrahydrate | | Sodium Perborate Tetrahydrate | | Sodium Perborate Tetrahydrate | | Sodium Perborate Tetrahydrate | | Sodium Perborate Tetrahydrate | | Sodium Perborate Tetrahydrate | | Sodium Perborate Tetrahydrate | |
| Treatment Dose | 0 | | 0.125 mg/kg | | 0.25 mg/kg | | 0.5 mg/kg | | 1 mg/kg | | 2 mg/kg | | 4 mg/kg | | 8 mg/kg/day | |
| Time of Sacrifice (in days) | 32 | | 32 | | 32 | | 32 | | 32 | | 32 | | 32 | | 32 | |
| #Rats/Group | 10 | | 10 | | 10 | | 10 | | 10 | | 10 | | 10 | | 10 | |

| **TISSUES** | No Exam | % | No Exam | % | No Exam | % | No Exam | % | No Exam | % | No Exam | % | No Exam | % | No Exam | % |
|---|---|---|---|---|---|---|---|---|---|---|---|---|---|---|---|---|
| **LUNG** | | | | | | | | | | | | | | | | |
| Within normal limits | 10/10 | 100 | 10/10 | 100 | 10/10 | 100 | 10/10 | 100 | 10/10 | 100 | 10/10 | 100 | 10/10 | 100 | 10/10 | 100 |
| Not examined | 0/10 | 0 | 0/10 | 0 | 0/10 | 0 | 0/10 | 0 | 0/10 | 0 | 0/10 | 0 | 0/10 | 0 | 0/10 | 0 |
| Notable or abnormal | 0/10 | 0 | 0/10 | 0 | 0/10 | 0 | 0/10 | 0 | 0/10 | 0 | 0/10 | 0 | 0/10 | 0 | 0/10 | 0 |
| Pulmonary edema | 0/10 | 0 | 0/10 | 0 | 0/10 | 0 | 0/10 | 0 | 0/10 | 0 | 0/10 | 0 | 0/10 | 0 | 0/10 | 0 |
| Lymphocytic infiltration | 0/10 | 0 | 0/10 | 0 | 0/10 | 0 | 0/10 | 0 | 0/10 | 0 | 0/10 | 0 | 0/10 | 0 | 0/10 | 0 |

| Group# | 1 | | 2 | | 3 | | 4 | | 5 | | 6 | | 7 | | 8 | |
|---|---|---|---|---|---|---|---|---|---|---|---|---|---|---|---|---|
| **LIVER** | | | | | | | | | | | | | | | | |
| Within normal limits | 9/10 | 90 | 10/10 | 100 | 10/10 | 100 | 10/10 | 100 | 10/10 | 100 | 10/10 | 100 | 10/10 | 100 | 10/10 | 100 |
| Not examined | 0/10 | 0 | 0/10 | 0 | 0/10 | 0 | 0/10 | 0 | 0/10 | 0 | 0/10 | 0 | 0/10 | 0 | 0/10 | 0 |
| Notable or abnormal | 1/10 | 10 | 0/10 | 0 | 0/10 | 0 | 0/10 | 0 | 0/10 | 0 | 0/10 | 0 | 0/10 | 0 | 0/10 | 0 |
| Fattening | 1/10 | 10 | 0/10 | 0 | 0/10 | 0 | 0/10 | 0 | 0/10 | 0 | 0/10 | 0 | 0/10 | 0 | 0/10 | 0 |
| Infiltration | 1/10 | 10 | 0/10 | 0 | 0/10 | 0 | 0/10 | 0 | 0/10 | 0 | 0/10 | 0 | 0/10 | 0 | 0/10 | 0 |

| **KIDNEY** | | | | | | | | | | | | | | | | |
|---|---|---|---|---|---|---|---|---|---|---|---|---|---|---|---|---|
| Within normal limits | 9/10 | 90 | 10/10 | 100 | 10/10 | 100 | 10/10 | 100 | 10/10 | 100 | 10/10 | 100 | 10/10 | 100 | 10/10 | 100 |
| Not examined | 0/10 | 0 | 0/10 | 0 | 0/10 | 0 | 0/10 | 0 | 0/10 | 0 | 0/10 | 0 | 0/10 | 0 | 0/10 | 0 |
| Notable or abnormal | 1/10 | 10 | 0/10 | 0 | 0/10 | 0 | 0/10 | 0 | 0/10 | 0 | 0/10 | 0 | 0/10 | 0 | 0/10 | 0 |
| Edema | 1/10 | 10 | 0/10 | 0 | 0/10 | 0 | 0/10 | 0 | 0/10 | 0 | 0/10 | 0 | 0/10 | 0 | 0/10 | 0 |

| **SPLEEN** | | | | | | | | | | | | | | | | |
|---|---|---|---|---|---|---|---|---|---|---|---|---|---|---|---|---|
| Within normal limits | 10/10 | 100 | 10/10 | 100 | 10/10 | 100 | 10/10 | 100 | 10/10 | 100 | 10/10 | 100 | 10/10 | 100 | 10/10 | 100 |
| Not examined | 0/10 | 0 | 0/10 | 0 | 0/10 | 0 | 0/10 | 0 | 0/10 | 0 | 0/10 | 0 | 0/10 | 0 | 0/10 | 0 |
| Notable or abnormal | 0/10 | 0 | 0/10 | 0 | 0/10 | 0 | 0/10 | 0 | 0/10 | 0 | 0/10 | 0 | 0/10 | 0 | 0/10 | 0 |

| Group# | 1 | | 2 | | 3 | | 4 | | 5 | | 6 | | 7 | | 8 | |
|---|---|---|---|---|---|---|---|---|---|---|---|---|---|---|---|---|
| **HEART** | | | | | | | | | | | | | | | | |
| Within normal limits | 10/10 | 100 | 10/10 | 100 | 10/10 | 100 | 10/10 | 100 | 10/10 | 100 | 10/10 | 100 | 10/10 | 100 | 10/10 | 100 |
| Not examined | 0/10 | 0 | 0/10 | 0 | 0/10 | 0 | 0/10 | 0 | 0/10 | 0 | 0/10 | 0 | 0/10 | 0 | 0/10 | 0 |
| Notable or abnormal | 0/10 | 0 | 0/10 | 0 | 0/10 | 0 | 0/10 | 0 | 0/10 | 0 | 0/10 | 0 | 0/10 | 0 | 0/10 | 0 |

| **PANCREAS** | | | | | | | | | | | | | | | | |
|---|---|---|---|---|---|---|---|---|---|---|---|---|---|---|---|---|
| Within normal limits | 10/10 | 100 | 10/10 | 100 | 10/10 | 100 | 10/10 | 100 | 10/10 | 100 | 10/10 | 100 | 10/10 | 100 | 10/10 | 100 |
| Not examined | 0/10 | 0 | 0/10 | 0 | 0/10 | 0 | 0/10 | 10 | 0/10 | 0 | 0/10 | 10 | 0/10 | 0 | 0/10 | 0 |
| Notable or abnormal | 0/10 | 0 | 0/10 | 0 | 0/10 | 0 | 0/10 | 0 | 0/10 | 0 | 0/10 | 0 | 0/10 | 0 | 0/10 | 0 |

| **INTESTINE, Large** | | | | | | | | | | | | | | | | |
|---|---|---|---|---|---|---|---|---|---|---|---|---|---|---|---|---|
| Within normal limits | 10/10 | 100 | 10/10 | 100 | 10/10 | 100 | 10/10 | 100 | 10/10 | 100 | 10/10 | 100 | 10/10 | 100 | 10/10 | 100 |
| Not examined | 0/10 | 0 | 0/10 | 0 | 0/10 | 0 | 0/10 | 0 | 0/10 | 0 | 0/10 | 0 | 0/10 | 0 | 0/10 | 0 |
| Notable or abnormal | 0/10 | 0 | 0/10 | 0 | 0/10 | 0 | 0/10 | 0 | 0/10 | 0 | 0/10 | 0 | 0/10 | 0 | 0/10 | 0 |

| Group# | 1 | | 2 | | 3 | | 4 | | 5 | | 6 | | 7 | | 8 | |
|---|---|---|---|---|---|---|---|---|---|---|---|---|---|---|---|---|
| **INTESTINE, Small** | | | | | | | | | | | | | | | | |
| Within normal limits | 10/10 | 100 | 10/1 0 | 100 | 10/10 | 100 | 10/10 | 100 | 10/10 | 100 | 10/10 | 100 | 10/10 | 100 | 9/10 | 90 |
| Not examined | 0/10 | 0 | 0/10 | 0 | 0/10 | 0 | 0/10 | 0 | 0/10 | 0 | 0/10 | 0 | 0/10 | 0 | 0/10 | 0 |
| Notable or abnormal | 0/10 | 0 | 0/10 | 0 | 0/10 | 0 | 0/10 | 0 | 0/10 | 0 | 0/10 | 0 | 0/10 | 0 | 1/10 | 10 |
| Enteritis | | | | | | | | | | | | | | | 1/10 | 10 |

| **STOMACH** | | | | | | | | | | | | | | | | |
|---|---|---|---|---|---|---|---|---|---|---|---|---|---|---|---|---|
| Within normal limits | 10/10 | 100 | 10/1 0 | 100 | 10/10 | 100 | 10/10 | 100 | 10/10 | 100 | 10/10 | 100 | 10/10 | 100 | 10/1 0 | 100 |
| Not examined | 0/10 | 0 | 0/10 | 0 | 0/10 | 0 | 0/10 | 0 | 0/10 | 0 | 0/10 | 0 | 0/10 | 0 | 0/10 | 0 |
| Notable or abnormal | 0/10 | 0 | 0/10 | 0 | 0/10 | 0 | 0/10 | 0 | 0/10 | 0 | 0/10 | 0 | 0/10 | 0 | 0/10 | 0 |

### Example 7 - comparison of perborate salts with boric acid and other boron derivatives

The cytoxic effect of boric acid, sodium pentaborate pentahydrate and sodium perborate tetrahydrate on small cell lung cancer cells (DMS114) was tested using the method described in Example 2 and the results are shown in Figures 23A-C. The EC50 (half maximal effective concentration; dose that kills 50% of cells in the population) is 1,000 µg/mL for boric acid, 1,250 µg/mL for sodium pentaborate pentahydrate and 15 µg/mL for sodium perborate tetrahydrate.

These examples show that the biological and cytotoxic effects of boric acid and other boron derivatives such as sodium pentaborate pentahydrate are not equivalent to the perborate salts such as sodium perborate tetrahydrate.

### Example 8 - perborate salts as combination treatments

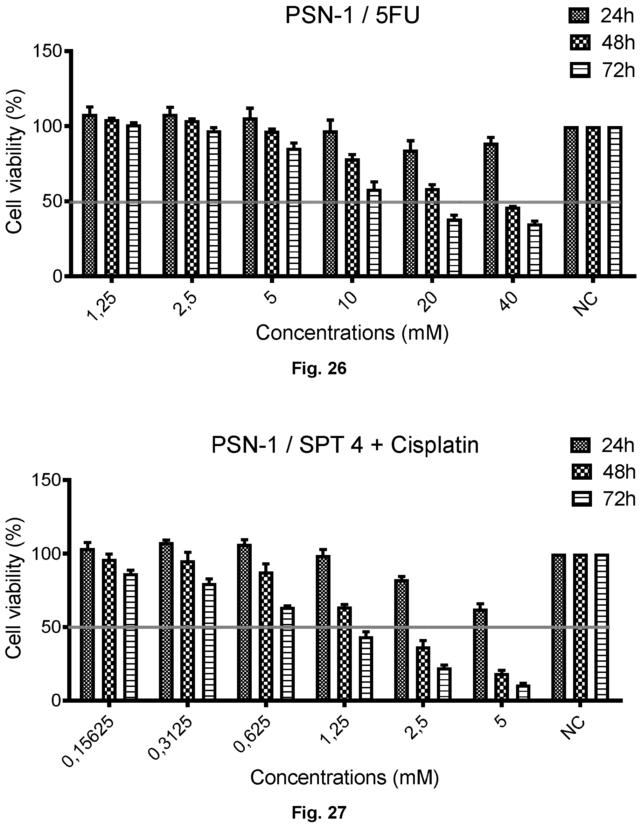

The cytoxic effect of sodium perborate tetrahydrate, Cisplatin, 5-Fluorouracil (5-FU), and their combinations on PSN-1 (a pancreatic adenocarcinoma cell line) was tested using the method described in Example 2. According to the cytotoxicity experiments, the EC50 concentration of sodium perborate tetrahydrate is 7.5 µg/mL (Figure 24), the EC50 concentration of Cisplatin is 2 mM (Figure 25), and the EC50 concentration of 5-Fluorouracil is 13 Mm (Figure 26).

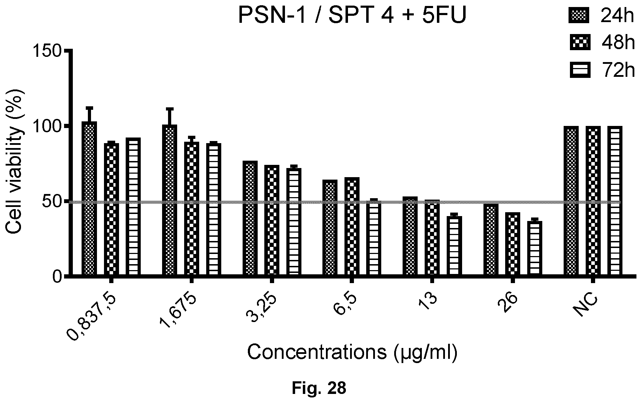

Surprisingly, it was found that when the chemotherapeutic agents Cisplatin and 5-FU were combined with a non-toxic dose of sodium perborate tetrahydrate (4 µg/mL), the EC50 value of Cisplatin decreased from 2 mM to 1 mM (Figure 27) and the EC50 value of 5-FU decreased from 13 mM to 6.5 mM (Figure 28). These results demonstrate that by combining sodium perborate tetrahydrate with one or more chemotherapy drugs, the effective concentration of the chemotherapy drug can be decreased.

Although various embodiments of the invention are disclosed herein, many adaptations and modifications may be made within the scope of the invention in accordance with the common general knowledge of those skilled in this art. Such modifications include the substitution of known equivalents for any aspect of the invention in order to achieve the same result in substantially the same way. Numeric ranges are inclusive of the numbers defining the range. The word "comprising" is used herein as an open-ended term, substantially equivalent to the phrase "including, but not limited to", and the word "comprises" has a corresponding meaning. As used herein, the singular forms "a", "an" and "the" include plural referents unless the context clearly dictates otherwise. Thus, for example, reference to "a thing" includes more than one such thing.

### References

Carrabba, Giorgio, Amir R. Dehdashti, and Fred Gentili. "Surgery for clival lesions: open resection versus the expanded endoscopic endonasal approach." Neurosurgical focus 25.6 (2008): E7.
Chugh, Rashmi, et al. "Chordoma: the nonsarcoma primary bone tumor." (2007).
Golden, Louis, and Juan E. Small. "Chordoma." Neuroradiology. Content Repository Only!, 2019. 226-232.
Hanahan, Douglas, and Robert A. Weinberg. "Hallmarks of cancer: the next generation." cell 144.5 (2011): 646-674.
Jackman, David M., and Bruce E. Johnson. "Small-cell lung cancer." The Lancet 366.9494 (2005): 1385-1396.
Lemjabbar-Alaoui, Hassan, et al. "Lung cancer: Biology and treatment options." Biochimica et Biophysica Acta (BBA)-Reviews on Cancer 1856.2 (2015): 189-210.
Murray, F. Jay. "A human health risk assessment of boron (boric acid and borax) in drinking water." Regulatory Toxicology and Pharmacology 22.3 (1995): 221-230.
Masetti, P. et al., "Cytotoxic potential of denture base and reline acrylic resins after immersion in disinfectant solutions", The Journal of Prosthetic Dentistry (2018)*.*
Dessordi, R. and Navarro, MA., "Boron action in bone health", Rheumatology and Orthopedic Medicine (2017) 2(1): 1-3
Meacham, S.L. et al., "Effect of boron supplementation on blood and urinary calcium, magnesium and phosphorus, and urinary boron in athletic and sedentary women", Am J Clin Nutr (1995) 61:241-5
Nana-Sinkam, Serge Patrick, and Charles A. Powell. "Molecular biology of lung cancer: Diagnosis and management of lung cancer: American College of Chest Physicians evidence-based clinical practice guidelines." Chest 143.5 (2013): e30S-e39S.
Simon, Ole, et al. "Pancreatic Cancer in the Year 2018-Room for Precision Medicine?." Deutsche medizinische Wochenschrift (1946) 143.15 (2018): 1109-1112. Zhang, Z. K., and Y. M. Yang. "Current research status and progress in comprehensive diagnosis and treatment of pancreatic cancer in the era of targeted therapy." Zhonghua wai ke za zhi [Chinese journal of surgery] 58.1 (2020): 22.
M. Bia 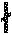 ek et al., "Selected physiological effects of boron compounds for animals and humans. A review", *Journal of Animal and Feed Sciences,* (2019): 307-320.
"Reproductive and General Toxicology of some Inorganic Borates and Risk Assessment for Human Beings", European Centre for Ecotoxicology and Toxicology of Chemicals (ECETOC) Technical Report No. 63 (1995), ISSN-0773-8072-63.
European Commission, Scientific Committee on Consumer Safety (SCCS), OPINION ON boron compounds, SCCS/1249/09 (2010).
European Commission, Scientific Committee on Consumer Safety (SCCS), OPINION ON sodium perborate and perboric acid, SCCS/1345/10 (2010).
Smith et al., "Large-scale human metabolic phenotyping and molecular epidemiological studies via 1H NMR spectroscopy of urine investigation of borate preservation", Anal Chem. 2009, 5;81(12):4847-56 - PMID: 19453167.
McKillop, A. and Sanderson, W. R., "Sodium perborate and sodium percarbonate: Cheap, safe and versatile oxidising agents for organic synthesis" Tetrahedron Volume 51, Issue 22, 29 May 1995, Pages 6145-6166.
Muzart, J., "Sodium Perborate and Sodium Percarbonate in Organic Synthesis", Thieme E-Journals, 1995 (DOI: 10.1055/s-1995-4128).
McKillop, A. and Sanderson, W.R., "Sodium perborate and sodium percarbonate: further applications in organic synthesis" Journal of the Chemical Society Perkin Transactions, 2000, 1 1(4):471-476 (doi:10.1039/A804579H).

## Claims

1. A perborate salt for use in a method for treating a cancer in a subject, the method comprising administering an effective amount of the perborate salt to the subject.

2. The perborate salt for use according to claim 1, wherein the cancer is a lung cancer, a pancreatic cancer, a chordoma, a breast cancer or an adenocarcinoma.

3. The perborate salt for use according to claim 1, wherein the cancer is small cell lung cancer.

4. The perborate salt for use according to any one of claims 1 to 3, wherein the perborate salt is a sodium perborate salt, a potassium perborate salt or a lithium perborate salt.

5. The perborate salt for use according to claim 4, wherein the perborate salt is NaBO₃·nH₂O (n = 1 to 6).

6. The perborate salt for use according to any one of claims 1 to 5, wherein the perborate salt is administered at a dose of at least about 0.05 mg/kg/day.

7. The perborate salt for use according to any one of claims 1 to 6, wherein the method further comprises administering a chemotherapy agent.

8. The perborate salt for use according to claim 7, wherein the chemotherapy agent comprises one or more of Altretamine; Bendamustine; Busulfan; Carboplatin; Carmustine; Chlorambucil; Cisplatin; Cyclophosphamide; Dacarbazine; Ifosfamide; Lomustine; Mechlorethamine; Chlormethine; Melphalan; Oxaliplatin; Temozolomide; Thiotepa; Trabectedin; Carmustine; Lomustine; Streptozocin; Azacitidine; 5-fluorouracil (5-FU); 6-mercaptopurine (6-MP); Capecitabine (Xeloda); Cladribine; Clofarabine; Cytarabine (Ara-C); Decitabine; Floxuridine; Fludarabine; Gemcitabine (Gemzar); Hydroxyurea; Methotrexate; Nelarabine; Pemetrexed (Alimta); Pentostatin; Pralatrexate; Thioguanine; Trifluridine/tipiracil combination; Daunorubicin; Doxorubicin; Epirubicin; Idarubicin; Valrubicin; Bleomycin; Dactinomycin; Mitomycin-C; Mitoxantrone; Irinotecan; Topotecan; Etoposide (VP-16); Mitoxantrone; Teniposide; Cabazitaxel; Docetaxel; Nab-paclitaxel; Paclitaxel; Vinblastine; Vincristine; Vinorelbine; Prednisone; Methylprednisolone; Dexamethasone; Arsenic trioxide; Asparaginase; Eribulin; Hydroxyurea; Ixabepilone; Mitotane; Omacetaxine; Pegaspargase; Procarbazine; Romidepsin; Vorinostat; or Raltitrexed.

9. The perborate salt for use according to any one of claims 1 to 8, wherein the perborate salt reduces gene expression of one or more genes in a cancer cell or a tumor cell in the subject, wherein the one or more genes are selected from the group consisting of PXN, VCL, TLN1, TNS2, RHOA, CDC42, KRAS, CCN2, C-Met and BIRC5.

10. The perborate salt for use according to any one of claims 1 to 8, wherein the perborate salt reduces gene expression of one or more genes in a cancer cell or a tumor cell in the subject, wherein the one or more genes are involved in a cell signalling pathway, wherein the cell signalling pathway is a cell cycle pathway, a DNA replication pathway, an EGF/EGFR signal pathway, a Focal Adhesion-Pl3K-AKT pathway, a focal adhesion pathway, a G protein signaling pathway, a Hippo-Merlin Signaling dysregulation pathway, a MAPK signaling pathway, an actin-cytoskeleton pathway, a TGF Beta signaling pathway and a WNT signaling pathway.

11. A pharmaceutical composition comprising an effective amount of a perborate salt for use according to any one of claims 1 to 6, 9 or 10 and a pharmaceutically acceptable carrier, diluent or adjuvant.

12. The pharmaceutical composition for use according to claim 11, wherein the pharmaceutical composition further comprises a chemotherapy agent.

13. The pharmaceutical composition for use according to claim 12, wherein the chemotherapy agent comprises one or more of Altretamine; Bendamustine; Busulfan; Carboplatin; Carmustine; Chlorambucil; Cisplatin; Cyclophosphamide; Dacarbazine; Ifosfamide; Lomustine; Mechlorethamine; Chlormethine; Melphalan; Oxaliplatin; Temozolomide; Thiotepa; Trabectedin; Carmustine; Lomustine; Streptozocin; Azacitidine; 5-fluorouracil (5-FU); 6-mercaptopurine (6-MP); Capecitabine (Xeloda); Cladribine; Clofarabine; Cytarabine (Ara-C); Decitabine; Floxuridine; Fludarabine; Gemcitabine (Gemzar); Hydroxyurea; Methotrexate; Nelarabine; Pemetrexed (Alimta); Pentostatin; Pralatrexate; Thioguanine; Trifluridine/tipiracil combination; Daunorubicin; Doxorubicin; Epirubicin; Idarubicin; Valrubicin; Bleomycin; Dactinomycin; Mitomycin-C; Mitoxantrone; Irinotecan; Topotecan; Etoposide (VP-16); Mitoxantrone; Teniposide; Cabazitaxel; Docetaxel; Nab-paclitaxel; Paclitaxel; Vinblastine; Vincristine; Vinorelbine; Prednisone; Methylprednisolone; Dexamethasone; Arsenic trioxide; Asparaginase; Eribulin; Hydroxyurea; Ixabepilone; Mitotane; Omacetaxine; Pegaspargase; Procarbazine; Romidepsin; Vorinostat; or Raltitrexed.

14. Use of a perborate salt in an *in vitro* method for reducing gene expression of one or more genes in a cancer cell or a tumor cell, the method comprising contacting the cell with an effective amount of the perborate salt, wherein the one or more genes are selected from the group consisting of PXN, VCL, TLN1, TNS2, RHOA, CDC42, KRAS, CCN2, C-Met and BIRC5.

15. Use of a perborate salt in an *in vitro* method for reducing gene expression of one or more genes in a cancer cell or a tumor cell, the method comprising contacting the cell with an effective amount of the perborate salt, wherein the one or more genes are involved in a cell signalling pathway, wherein the cell signalling pathway is a cell cycle pathway, a DNA replication pathway, an EGF/EGFR signal pathway, a Focal Adhesion-Pl3K-AKT pathway, a focal adhesion pathway, a G protein signaling pathway, a Hippo-Merlin Signaling dysregulation pathway, a MAPK signaling pathway, an actin-cytoskeleton pathway, a TGF Beta signaling pathway and a WNT signaling pathway.

## Patentansprüche

1. Perboratsalz zur Verwendung in einem Verfahren zur Behandlung eines Krebses in einem Subjekt, wobei das Verfahren die Verabreichung einer wirksamen Menge des Perboratsalzes an das Subjekt umfasst.

2. Perboratsalz zur Verwendung nach Anspruch 1, wobei der Krebs ein Lungenkrebs, ein Bauchspeicheldrüsenkrebs, ein Chordom, ein Brustkrebs oder ein Adenokarzinom ist.

3. Perboratsalz zur Verwendung nach Anspruch 1, wobei der Krebs kleinzelliger Lungenkrebs ist.

4. Perboratsalz zur Verwendung nach einem der Ansprüche 1 bis 3, wobei das Perboratsalz ein Natriumperboratsalz, ein Kaliumperboratsalz oder ein Lithiumperboratsalz ist.

5. Perboratsalz zur Verwendung nach Anspruch 4, wobei das Perboratsalz NaBO₃·nH₂O (n = 1 bis 6) ist.

6. Perboratsalz zur Verwendung nach einem der Ansprüche 1 bis 5, wobei das Perboratsalz in einer Dosis von mindestens etwa 0,05 mg/kg/Tag verabreicht wird.

7. Perboratsalz zur Verwendung nach einem der Ansprüche 1 bis 6, wobei das Verfahren weiter die Verabreichung eines Chemotherapeutikums umfasst.

8. Perboratsalz zur Verwendung nach Anspruch 7, wobei das Chemotherapeutikum eines oder mehrere umfasst von Altretamin; Bendamustin; Busulfan; Carboplatin; Carmustin; Chlorambucil; Cisplatin; Cyclophosphamid; Dacarbazin; Ifosfamid; Lomustin; Mechlorethamin; Chlormethin; Melphalan; Oxaliplatin; Temozolomid; Thiotepa; Trabectedin; Carmustin; Lomustin; Streptozocin; Azacitidin; 5-Fluorouracil (5-FU); 6-Mercaptopurin (6-MP); Capecitabin (Xeloda); Cladribin; Clofarabin; Cytarabin (Ara-C); Decitabin; Floxuridin; Fludarabin; Gemcitabin (Gemzar); Hydroxyharnstoff; Methotrexat; Nelarabin; Pemetrexed (Alimta); Pentostatin; Pralatrexat; Thioguanin; Trifluridin/Tipiracil Kombination; Daunorubicin; Doxorubicin; Epirubicin; Idarubicin; Valrubicin; Bleomycin; Dactinomycin; Mitomycin-C; Mitoxantron; Irinotecan; Topotecan; Etoposid (VP-16); Mitoxantron; Teniposid; Cabazitaxel; Docetaxel; Nab-Paclitaxel; Paclitaxel; Vinblastin; Vincristin; Vinorelbin; Prednison; Methylprednisolon; Dexamethason; Arsentrioxid; Asparaginase; Eribulin; Hydroxyharnstoff; Ixabepilon; Mitotan; Omacetaxin; Pegaspargase; Procarbazin; Romidepsin; Vorinostat; oder Raltitrexed.

9. Perboratsalz zur Verwendung nach einem der Ansprüche 1 bis 8, wobei das Perboratsalz die Genexpression eines oder mehrerer Gene in einer Krebszelle oder einer Tumorzelle in dem Subjekt verringert, wobei das eine oder die mehreren Gene ausgewählt sind aus der Gruppe bestehend aus PXN, VCL, TLN1, TNS2, RHOA, CDC42, KRAS, CCN2, C-Met und BIRC5.

10. Perboratsalz zur Verwendung nach einem der Ansprüche 1 bis 8, wobei das Perboratsalz die Genexpression eines oder mehrerer Gene in einer Krebszelle oder einer Tumorzelle in dem Subjekt verringert, wobei das eine oder die mehreren Gene an einem Zellsignalweg beteiligt sind, wobei es sich bei dem Zellsignalweg um einen Zellzyklusweg, einen DNA-Replikationsweg, einen EGF/EGFR-Signalweg, einen Focal Adhesion-PI3K-AKT-Weg, einen fokalen Adhäsionsweg, einen G-Protein-Signalweg, einen Hippo-Merlin-Signaldysregulationsweg, einen MAPK-Signalweg, einen Aktin-Zytoskelett-Weg, einen TGF-Beta-Signalweg und einen WNT-Signalweg handelt.

11. Pharmazeutische Zusammensetzung, umfassend eine wirksame Menge eines Perboratsalzes zur Verwendung nach einem der Ansprüche 1 bis 6, 9 oder 10 und einen pharmazeutisch annehmbaren Träger, ein pharmazeutisch annehmbares Verdünnungsmittel oder einen pharmazeutisch annehmbaren Hilfsstoff.

12. Pharmazeutische Zusammensetzung zur Verwendung nach Anspruch 11, wobei die pharmazeutische Zusammensetzung weiter ein Chemotherapeutikum umfasst.

13. Pharmazeutische Zusammensetzung zur Verwendung nach Anspruch 12, wobei das Chemotherapeutikum eines oder mehrere umfasst von Altretamin; Bendamustin; Busulfan; Carboplatin; Carmustin; Chlorambucil; Cisplatin; Cyclophosphamid; Dacarbazin; Ifosfamid; Lomustin; Mechlorethamin; Chlormethin; Melphalan; Oxaliplatin; Temozolomid; Thiotepa; Trabectedin; Carmustin; Lomustin; Streptozocin; Azacitidin; 5-Fluorouracil (5-FU); 6-Mercaptopurin (6-MP); Capecitabin (Xeloda); Cladribin; Clofarabin; Cytarabin (Ara-C); Decitabin; Floxuridin; Fludarabin; Gemcitabin (Gemzar); Hydroxyharnstoff; Methotrexat; Nelarabin; Pemetrexed (Alimta); Pentostatin; Pralatrexat; Thioguanin; Trifluridin/Tipiracil Kombination; Daunorubicin; Doxorubicin; Epirubicin; Idarubicin; Valrubicin; Bleomycin; Dactinomycin; Mitomycin-C; Mitoxantron; Irinotecan; Topotecan; Etoposid (VP-16); Mitoxantron; Teniposid; Cabazitaxel; Docetaxel; Nab-Paclitaxel; Paclitaxel; Vinblastin; Vincristin; Vinorelbin; Prednison; Methylprednisolon; Dexamethason; Arsentrioxid; Asparaginase; Eribulin; Hydroxyharnstoff; Ixabepilon; Mitotan; Omacetaxin; Pegaspargase; Procarbazin; Romidepsin; Vorinostat; oder Raltitrexed.

14. Verwendung eines Perboratsalzes in einem *in vitro*-Verfahren zur Verringerung der Genexpression eines oder mehrerer Gene in einer Krebszelle oder einer Tumorzelle, wobei das Verfahren das Inkontaktbringen der Zelle mit einer wirksamen Menge des Perboratsalzes umfasst, wobei das eine oder die mehreren Gene ausgewählt sind aus der Gruppe bestehend aus PXN, VCL, TLN1, TNS2, RHOA, CDC42, KRAS, CCN2, C-Met und BIRC5.

15. Verwendung eines Perboratsalzes in einem *in vitro*-Verfahren zur Verringerung der Genexpression eines oder mehrerer Gene in einer Krebszelle oder einer Tumorzelle, wobei das Verfahren das Inkontaktbringen der Zelle mit einer wirksamen Menge des Perboratsalzes umfasst, wobei das eine oder die mehreren Gene an einem Zellsignalweg beteiligt sind, wobei es sich bei dem Zellsignalweg um einen Zellzyklusweg, einen DNA-Replikationsweg, einen EGF/EGFR-Signalweg, einen Focal Adhesion-PI3K-AKT-Weg, einen fokalen Adhäsionsweg, einen G-Protein-Signalweg, einen Hippo-Merlin-Signaldysregulationsweg, einen MAPK-Signalweg, einen Aktin-Zytoskelett-Weg, einen TGF-Beta-Signalweg und einen WNT-Signalweg handelt.

## Revendications

1. Sel de perborate pour utilisation dans un procédé pour traiter un cancer chez un sujet, le procédé comprenant l'administration chez le sujet d'une quantité efficace du sel de perborate.

2. Sel de perborate pour utilisation selon la revendication 1, dans lequel le cancer est un cancer du poumon, un cancer du pancréas, un chordome, un cancer du sein ou un adénocarcinome.

3. Sel de perborate pour utilisation selon la revendication 1, dans lequel le cancer est un cancer du poumon à petites cellules.

4. Sel de perborate pour utilisation selon l'une quelconque des revendications 1 à 3, dans lequel le sel de perborate est un sel de perborate de sodium, un sel de perborate de potassium ou un sel de perborate de lithium.

5. Sel de perborate pour utilisation selon la revendication 4, dans lequel le sel de perborate est NaBO₃·nH₂O (n = 1 à 6).

6. Sel de perborate pour utilisation selon l'une quelconque des revendications 1 à 5, dans lequel le sel de perborate est administré à une dose d'au moins environ 0,05 mg/kg/jour.

7. Sel de perborate pour utilisation selon l'une quelconque des revendications 1 à 6, dans lequel le procédé comprend en outre l'administration d'un agent chimiothérapeutique.

8. Sel de perborate pour utilisation selon la revendication 7, dans lequel l'agent chimiothérapeutique comprend un ou plusieurs agents parmi : altrétamine ; bendamustine ; busulfan ; carboplatine ; carmustine ; chlorambucil ; cisplatine ; cyclophosphamide ; dacarbazine ; ifosfamide ; lomustine ; méchloréthamine ; chlorméthine ; melphalan ; oxaliplatine ; témozolomide ; thiotépa ; trabectédine ; carmustine ; lomustine ; streptozocine ; azacitidine ; 5-fluorouracile (5-FU) ; 6-mercaptopurine (6-MP) ; capécitabine (Xeloda) ; cladribine ; clofarabine ; cytarabine (Ara-C) ; décitabine ; floxuridine ; fludarabine ; gemcitabine (Gemzar) ; hydroxyurée ; méthotrexate ; nélarabine ; pémétrexed (Alimta) ; pentostatine ; pralatrexate ; thioguanine ; combinaison trifluridine/tipiracil ; daunorubicine ; doxorubicine ; épirubicine ; idarubicine ; valrubicine ; bléomycine ; dactinomycine ; mitomycine-C ; mitoxantrone ; irinotécan ; topotécan ; étoposide (VP-16) ; mitoxantrone ; téniposide ; cabazitaxel ; docétaxel ; nab-paclitaxel ; paclitaxel ; vinblastine ; vincristine ; vinorelbine ; prednisone ; méthylprednisolone ; dexaméthasone ; trioxyde d'arsenic ; asparaginase ; éribuline ; hydroxyurée ; ixabépilone ; mitotane ; omacétaxine ; pégaspargase ; procarbazine ; romidepsine ; vorinostat ; ou raltitrexed.

9. Sel de perborate pour utilisation selon l'une quelconque des revendications 1 à 8, dans lequel le sel de perborate réduit l'expression génique d'un ou plusieurs gênes dans une cellule cancéreuse ou une cellule tumorale chez le sujet, dans lequel les un ou plusieurs gênes sont sélectionnés dans le groupe constitué de PXN, VCL, TLN1, TNS2, RHOA, CDC42, KRAS, CCN2, C-Met et BIRC5.

10. Sel de perborate pour utilisation selon l'une quelconque des revendications 1 à 8, dans lequel le sel de perborate réduit l'expression génique d'un ou plusieurs gênes dans une cellule cancéreuse ou une cellule tumorale chez le sujet, dans lequel les un ou plusieurs gênes sont impliqués dans une voie de signalisation cellulaire, dans lequel la voie de signalisation cellulaire est une voie de cycle cellulaire, une voie de réplication d'ADN, une voie de signal EGF/EGFR, une voie PI3K-AKT d'adhérence focale, une voie d'adhérence focale, une voie de signalisation de la protéine G, une voie de dérégulation de signalisation Hippo-Merlin, une voie de signalisation MAPK, une voie actine-cytosquelette, une voie de signalisation TGF Beta et une voie de signalisation WNT.

11. Composition pharmaceutique comprenant une quantité efficace d'un sel de perborate destinée à une utilisation selon l'une quelconque des revendications 1 à 6, 9 ou 10 et un vecteur, diluant ou adjuvant pharmaceutiquement acceptable.

12. Composition pharmaceutique destinée à une utilisation selon la revendication 11, dans laquelle la composition pharmaceutique comprend en outre un agent chimiothérapeutique.

13. Composition pharmaceutique destinée à une utilisation selon la revendication 12, dans laquelle l'agent chimiothérapeutique comprend un ou plusieurs agents parmi : altrétamine ; bendamustine ; busulfan ; carboplatine ; carmustine ; chlorambucil ; cisplatine ; cyclophosphamide ; dacarbazine ; ifosfamide ; lomustine ; méchloréthamine ; chlorméthine ; melphalan ; oxaliplatine ; témozolomide ; thiotépa ; trabectédine ; carmustine ; lomustine ; streptozocine ; azacitidine ; 5-fluorouracile (5-FU) ; 6-mercaptopurine (6-MP) ; capécitabine (Xeloda) ; cladribine ; clofarabine ; cytarabine (Ara-C) ; décitabine ; floxuridine ; fludarabine ; gemcitabine (Gemzar) ; hydroxyurée ; méthotrexate ; nélarabine ; pémétrexed (Alimta) ; pentostatine ; pralatrexate ; thioguanine ; combinaison trifluridine/tipiracil ; daunorubicine ; doxorubicine ; épirubicine ; idarubicine ; valrubicine ; bléomycine ; dactinomycine ; mitomycine-C ; mitoxantrone ; irinotécan ; topotécan ; étoposide (VP-16) ; mitoxantrone ; téniposide ; cabazitaxel ; docétaxel ; nab-paclitaxel ; paclitaxel ; vinblastine ; vincristine ; vinorelbine ; prednisone ; méthylprednisolone ; dexaméthasone ; trioxyde d'arsenic ; asparaginase ; éribuline ; hydroxyurée ; ixabépilone ; mitotane ; omacétaxine ; pégaspargase ; procarbazine ; romidepsine ; vorinostat ; ou raltitrexed.

14. Utilisation d'un sel de perborate dans un procédé *in vitro* pour réduire l'expression génique d'un ou plusieurs gênes dans une cellule cancéreuse ou une cellule tumorale, le procédé comprenant la mise en contact de la cellule avec une quantité efficace du sel de perborate, dans lequel les un ou plusieurs gênes sont sélectionnés dans le groupe constitué de PXN, VCL, TLN1, TNS2, RHOA, CDC42, KRAS, CCN2, C-Met et BIRC5.

15. Utilisation d'un sel de perborate dans un procédé *in vitro* pour réduire l'expression génique d'un ou plusieurs gênes dans une cellule cancéreuse ou une cellule tumorale, le procédé comprenant la mise en contact de la cellule avec une quantité efficace du sel de perborate, dans lequel les un ou plusieurs gênes sont impliqués dans une voie de signalisation cellulaire, dans lequel la voie de signalisation cellulaire est une voie de cycle cellulaire, une voie de réplication d'ADN, une voie de signal EGF/EGFR, une voie PI3K-AKT d'adhérence focale, une voie d'adhérence focale, une voie de signalisation de la protéine G, une voie de dérégulation de signalisation Hippo-Merlin, une voie de signalisation MAPK, une voie actine-cytosquelette, une voie de signalisation TGF Beta et une voie de signalisation WNT.
